# EUROPEAN PATENT SPECIFICATION

(11) **EP 3 015 476 B1**
(45) Date of publication and mention of the grant of the patent: **10.04.2019**
(21) Application number: 14817679.5
(22) Date of filing: 27.06.2014
(51) Int. Cl.: C07K 14/435, G01N 33/574, G01N 33/577

(54) **HSP90ALPHA AS TUMOR BIOMARKER**
HSP90ALPHA ALS TUMORBIOMARKER
HSP90ALPHA COMME BIOMARQUEUR TUMORAL

(30) Priority: 27.06.2013 CN 201310264285; 15.11.2013 CN 201310587369
(43) Date of publication of application: 04.05.2016
(73) Proprietor: Tsinghua University, Haidian District Beijing 100084 (CN); Yantai Protgen Biotechnology Development Co. Ltd., Shandong 264006 (CN); Beijing Protgen Ltd., Beijing 100085 (CN)
(72) Inventor: LUO, Yongzhang, Beijing 100084 (CN); CUI, Dawei, Yantai Shandong 264006 (CN); FU, Yan, Beijing 100084 (CN); WU, Fei, Beijing 100085 (CN); SONG, Xiaomin, Beijing 100084 (CN); LI, Michuan, Beijing 100085 (CN); CHANG, Guodong, Beijing 100085 (CN); LU, Chuntao, Beijing 100085 (CN)
(74) Representative: Manitz Finsterwald Patent- und Rechtsanwaltspartnerschaft mbB
(86) International application number: PCT/CN2014/081034
(87) International publication number: WO 2014/206353

(56) References cited:
- EP-A1- 2 452 948
- WO-A1-2013/028907
- WO-A2-2008/070472
- CN-A- 101 514 989
- CN-A- 101 942 017
- US-A1- 2010 129 829
- BURGESS EARLE F ET AL: "Prostate cancer serum biomarker discovery through proteomic analysis of alpha-2 macroglobulin protein complexes", PROTEOMICS - CLINICAL APPLICATIONS, WILEY, DE, vol. 2, no. 9, 1 September 2008 (2008-09-01), pages 1223-1233, XP009135871, ISSN: 1862-8346
- J.-S. CHEN ET AL: "Secreted Heat Shock Protein 90 Induces Colorectal Cancer Cell Invasion through CD91/LRP-1 and NF- B-mediated Integrin V Expression", JOURNAL OF BIOLOGICAL CHEMISTRY, vol. 285, no. 33, 13 August 2010 (2010-08-13), pages 25458-25466, XP055321576, US ISSN: 0021-9258, DOI: 10.1074/jbc.M110.139345
- Y. SHI ET AL: "Plasma Levels of Heat Shock Protein 90 Alpha Associated with Lung Cancer Development and Treatment Responses", CLINICAL CANCER RESEARCH, vol. 20, no. 23, 14 October 2014 (2014-10-14), pages 6016-6022, XP055321586, US ISSN: 1078-0432, DOI: 10.1158/1078-0432.CCR-14-0174
- Song Zuozuo ET AL: "Analysis of affecting factors in the detection of tumor patients heat shock protein 90[alpha] using ELISA", Chinese Journal of Laboratory Medicine, 1 December 2013 (2013-12-01), page Abstract, XP055321531, Retrieved from the Internet: URL:http://caod.oriprobe.com/articles/4085 9549/Analysis_of_affecting_factors_in_the_ detection_of_tumor_patients_heat_.htm [retrieved on 2016-11-22]
- KINGA MUSIAL ET AL: "Heat shock proteins in children and young adults on chronic hemodialysis", PEDIATRIC NEPHROLOGY ; JOURNAL OF THE INTERNATIONAL PEDIATRIC NEPHROLOGY ASSOCIATION, SPRINGER, BERLIN, DE, vol. 24, no. 10, 28 May 2009 (2009-05-28), pages 2029-2034, XP019741250, ISSN: 1432-198X, DOI: 10.1007/S00467-009-1197-7
- W. GOOSSENS ET AL: "K2- or K3-EDTA: the anticoagulant of choice in routine haematology?", CLINICAL AND LABORATORY HAEMATOLOGY, vol. 13, no. 3, 1 September 1991 (1991-09-01), pages 291-295, XP055365060, OXFORD ISSN: 0141-9854, DOI: 10.1111/j.1365-2257.1991.tb00284.x

## Description

### Field of the Invention

The present invention relates to a method of determining whether a subject is suffering from a cancer or has a risk of cancer, including detecting the concentration of Hsp90α (heat shock protein 90α) in a plasma sample from the subject. More particularly, the present invention relates to a method of diagnosing lung cancer, liver cancer, colorectal cancer or breast cancer, by quantitative detection of heat shock protein 90α. The present invention also relates to a kit for determining whether a subject is suffering from a cancer or has a risk of cancer.

### Background of the Invention

Heat shock protein 90α (Hsp90α) is an abundant intracellular chaperone protein. Recent studies reported that Hsp90α may be secreted into extracellular space, which is closely related with the occurrence and development of cancer. We have proved that the Hsp90α secreted into the peripheral blood may be conveniently quantified and the concentration thereof is relevant to tumor burden and malignancy, but there is no data to guide the application of this indicator in clinical use. Therefore, how to make use of secreted Hsp90α in tumor clinical practice is an urgent problem to be resolved.

Patent application EP 2 452 948 A1 (published May 16, 2012)generally relates to the diagnosis and treatment of cancer and describes a method of diagnosing the presence or metastasis of cancer by detecting plasma Hsp90α having a specific amino acid sequence. However, no practical or specific steps of the method have been disclosed, thus, there is an utmost need for the exploration of the envisaged method.

### Summary of the Invention

In one aspect, the present invention provides a method of determining whether a subject is suffering from a cancer or has a risk of cancer, including detecting the concentration of Hsp90α (heat shock protein 90α) in a plasma sample from the subject, wherein if the concentration reaches or exceeds a pre-set threshold, it is determined that the subject is suffering from a cancer or has a risk of cancer; wherein the threshold is selected from the range of 50 ng/ml to 120 ng/ml, and wherein the concentration of Hsp90α is detected using EDTA-K2 as an anticoagulant.

In a particular embodiment of the invention, the method further comprises detecting the concentration of at least one other tumor biomarker in said sample, wherein if the concentration of Hsp90α and the at least one other tumor biomarker reaches or exceeds a pre-set threshold, it is determined that the subject is suffering from a cancer or has a risk of cancer.

In another embodiment of the invention, the threshold is selected from the group consisting of 50, 53, 56, 62, 63, 64, 67, 72, 82, 85, and 117 ng/ml.

In another embodiment of the invention, the threshold is 82 ng/ml.

In another embodiment of the invention, the cancer is selected from the group consisting of lung cancer, liver cancer, colorectal cancer, and breast cancer.

In another aspect, the present invention provides a kit for determining whether a subject is suffering from a cancer or has a risk of cancer including: a monoclonal antibody against human secretory Hsp90α, a detection reagent, a container for receiving a blood sample, wherein the container comprises EDTA-K2 as an anticoagulant, and instructions, wherein the instructions indicate the threshold of Hsp90α concentration in a plasma sample of a healthy human, and wherein if the Hsp90α concentration in a plasma sample from the subject reaches or exceeds the threshold, the subject is determined to have a cancer or have a risk of cancer; wherein the threshold is selected from the range of 50 ng/ml to 120 ng/ml, and wherein the concentration of Hsp90α is detected using EDTA-K2 as an anticoagulant.

### Summary of other aspects of the Disclosure

In one aspect, the present disclosure provides a method of determining whether a subject is suffering from a cancer or has a risk of cancer, including detecting the concentration of Hsp90α in a blood sample from the subject, wherein if the concentration reaches or exceeds a preset threshold, it is determined that the subject is suffering from a cancer or has a risk of cancer. The threshold is selected from the range of 50 ng/ml to 120 ng/ml, for example, the threshold may be 50, 53, 56, 62, 63, 64, 67, 72, 82, 85, or 117 ng/ml, and values within ±15% around the threshold are considered to have equal significance for determination.

A skilled artisan would understand that it is quite difficult to obtain a completely accurate concentration due to the limitation of the detection methods. According to the general practice in the field, variations within ±15% are considered to be reasonable errors. For example, when the threshold value is set at 50 ng/ml, if the detected Hsp90α concentration of a subject is 43 ng/ml, the subject would still be determined to be a cancer patient or have a high risk of cancer.

In the present disclosure, the blood sample may be a plasma or serum sample. In a preferred embodiment of the invention, plasma samples are used, due to their higher measurement accuracy.

The threshold of Hsp90α may be 50, 53, 56, 62, 63, 64, 67, 72, 82, 85 or 117 ng/ml. For example, in a particular aspect of the disclosure, a particularly preferred threshold value for lung cancer is 67.47 ng/ml (which may be rounded to 67 ng/ml), and the corresponding specificity is 85%, corresponding sensitivity is 64%. Values within ±15% around the aforementioned threshold may be considered as equivalent for determination.

In a particular aspect of the disclosure, a preferred threshold for lung cancer is 82 ng/ml, namely concentrations of Hsp90α in the range of 0-82 ng/ml are considered to be normal. In a preferred aspect of the present disclosure, a particularly preferred threshold for lung cancer is 53 ng/ml, namely concentrations of Hsp90α in the range of 0-53 ng/ml are considered to be normal. Similarly, values within ±15% around the threshold may be considered to have equivalent significance for determination. Preferably, the blood sample is a plasma sample.

In another aspect, the present disclosure provides a method for disease monitoring and/or treatment efficacy evaluation of a cancer patient who is receiving a treatment, or for the efficacy evaluation of a candidate drug, comprising detecting the Hsp90α concentration in a blood sample of the subject before and after the treatment. If the percentage of increase of Hsp90α concentration after treatment reaches or exceeds a predetermined threshold in comparison to the concentration of Hsp90α before the treatment, it is determined that disease progresses in the subject and/or the efficacy of the treatment is poor, or the efficacy of the candidate drug is poor. Wherein said threshold is selected from the range of 10% to 50%. For example, the threshold may be 34%, and values within ±15% around the threshold may be considered to have equivalent significance for determination. Preferably, the blood sample is a plasma sample.

The term "treatment" used herein should be understood in a broad meaning. For example, the therapy of a lung cancer patient may involve multiple treatment cycles, such as 8 treatment cycles in total. If taking the entire therapeutic process as a whole, the Hsp90α concentration may be detected before the start of the first cycle and at the end of the eighth cycle, respectively, to evaluate the efficacy of the entire therapy. Alternatively, each treatment cycle may be considered as a single "therapy" (for the purpose of getting a more accurate assessment). For example, Hsp90α concentration may be detected before and after each treatment cycle, and thereby the efficacy of a single treatment cycle may be evaluated. Even more accurate assessment may be carried out. For example, for a 20-day treatment cycle, each day may be considered as a single "treatment", and concentrations of Hsp90α may be detected before and after each daily treatment to evaluate the therapeutic efficacy of that day. A skilled artisan may set the specific time span of a "treatment" according to different evaluation requirements.

The present disclosure also provides a method of determining whether a subject is suffering from a cancer or has a risk of cancer, including detecting the concentration of Hsp90α and at least one other tumor biomarker in a blood sample of the subject. If the concentration of Hsp90α and the other tumor biomarker reaches or exceeds a predetermined threshold, it is determined that the subject is suffering from a cancer or has a risk of cancer, wherein the threshold of Hsp90α is selected from the range of 50 ng/ml to 120 ng/ml, such as 50, 53, 56, 62, 63, 64, 67, 72, 82, 85 or 117 ng/ml. Values within ±15% of the threshold are considered to have equivalent significance for determination. Preferably, the blood sample is a plasma sample. The other tumor biomarker is selected from the group consisting of carcinoembryonic antigen (CEA), cytokeratin-19 fragment antigen 21-1 (CYFRA21-1), AFP, CA19-9, CA125, CA15-3, PSA and CA72-4.

According to a preferred aspect of the present disclosure, EDTA-K2 is a preferred anticoagulant in the detection of Hsp90α concentration.

The method of the present disclosure is applicable to the following cancer types: lung cancer, liver cancer, colorectal cancer, breast cancer, gastric cancer, pancreatic cancer, ovarian cancer, lymphoma, esophageal cancer, melanoma, kidney cancer, uterine cancer, nasopharyngeal carcinoma, osteosarcoma, bladder cancer and prostate cancer.

The present disclosure also provides a kit for determining whether a subject is suffering from a cancer or has a risk of cancer, comprising: an optional secretory human Hsp90α, a monoclonal antibody against secretory human Hsp90α, a detection reagent, and instructions, wherein the instructions explicitly indicate the threshold of Hsp90α concentration in a blood sample of a healthy human. If the Hsp90α concentration of the subject reaches or exceeds the threshold value, it is determined that the subject is suffering from a cancer or has a risk of cancer, wherein said threshold value is selected from 50 ng/ml to 120 ng/ml, such as 50, 53, 56, 62, 63, 64, 67, 72, 82, 85 or 117 ng/ml. Values within ±15% around the threshold may be considered to have equivalent significance for determination. Preferably, the blood sample is a plasma sample.

The term "optional" as used herein means "may have" or "may not have". For example, "optional secretory human Hsp90α" means the kit of the present disclosure may include secretory human Hsp90α or may not include secretory human Hsp90α. For example, the secretory human Hsp90α may be packaged separately and used in combination with the main part of the kit. Preferably, the kit of the present disclosure may contain secretory human Hsp90α as detection standard for convenience of use. Recombinant secretory human Hsp90α is particularly preferred.

According to a preferred aspect of the present disclosure, the kit may include a container for receiving a blood sample, and the container comprises EDTA-K2. In a preferred aspect of the present disclosure, the container for receiving a blood sample is a blood collection tube comprising EDTA-K2. According to an aspect of the present disclosure, EDTA-K2 is a preferred anticoagulant in the detection of Hsp90α concentration.

The kit of the present disclosure is applicable to the following cancer types: lung cancer, liver cancer, colorectal cancer, breast cancer, gastric cancer, pancreatic cancer, ovarian cancer, lymphoma, esophageal cancer, melanoma, kidney cancer, uterine cancer, nasopharyngeal carcinoma, osteosarcoma tumors, bladder cancer and prostate cancer.

In examples of the present disclosure, the performance of Hsp90α quantitative detection in cancer auxiliary diagnosis and therapeutic efficacy monitoring are evaluated in multiple types of cancer. It is found that the threshold of Hsp90α concentration in cancer diagnosis is applicable to lung cancer, liver cancer, colorectal cancer, breast cancer, and other types of cancer. Detailed statistical parameters for the use of quantitative detection of tumor biomarker Hsp90α in the diagnosis of lung cancer, liver cancer, colorectal cancer and breast cancer are also provided.

In examples of the present disclosure, corresponding ROC curves were drawn based on the data from different groups of people. According to well-known technique in this field, each ROC curve provides a series of thresholds and corresponding sensitivity and specificity. Thus, from these ROC curves, a skilled artisan may easily check out the diagnosis performance of the detection based on a given threshold value (also known as cut-off value), which includes the sensitivity, specificity and accuracy of the diagnosis in corresponding cancer type.

For example, for liver cancer, when the cut-off value of cancer patients vs. healthy people is 52.79 ng/ml, the sensitivity, specificity, and accuracy are 93.7%, 85%, and 94.5%, respectively; when the cut-off value is 62.20 ng/ml, the sensitivity, specificity, and accuracy are 91.4%, 90%, and 92.2%; when the cut-off value of cancer patients vs. non-cancerous liver disease patients is 63.66 ng/ml, the sensitivity, specificity, and accuracy are 90.7%, 90%, and 90.3%, respectively; when the cut-off value is 85.24 ng/ml, the sensitivity, specificity, and accuracy are 80.7%, 95%, and 88.6%, respectively.

For colorectal cancer, when the cut-off value of cancer patients vs. healthy people and non-cancerous colorectal disease patients is 52.79 ng/ml, the sensitivity, specificity, and accuracy are 89.9%, 85%, and 87%, respectively; when the cut-off value is 62.20 ng/ml, the sensitivity, specificity, and accuracy are 83.4%, 90%, and 87%, respectively.

For breast cancer, when the cut-off value of cancer patients vs. healthy people is 52.66 ng/ml, the sensitivity, specificity, and accuracy are 65.6%, 85%, and 73.85%, respectively; when the cut-off value is 61.92 ng/ml, the sensitivity, specificity, and accuracy are 54.6%, 90%, and 69.68%; when the cut-off value of cancer patients vs. non-cancerous breast disease patients is 63.41 ng/ml, the sensitivity, specificity, and accuracy are 52.4%, 84.9%, and 68.29%, respectively; when the cut-off value is 72.21 ng/ml, the sensitivity, specificity, and accuracy are 41.9%, 90.1%, and 65.41%, respectively.

A skilled artisan would understand that the cut-off value and the corresponding sensitivity, specificity and accuracy might be different due to different subjects and cancer types, but are included in the present disclosure. According to the requirements for specific tumor type, sensitivity and specificity, a skilled artisan may choose an appropriate optimal threshold from the ROC curve or from the threshold range disclosed in the present disclosure.

The present disclosure also verified that the plasma Hsp90α concentration in lung cancer, liver cancer, colorectal cancer, and breast cancer patients after surgery was significantly decreased as compared with that before surgery. Thus, this index may be used in the evaluation of surgical treatment efficacy. In addition, in the patients who obtained clinical benefit (CR+PR+SD) from a medical treatment, the Hsp90α concentration was significantly decreased after the treatment as compared with that before the treatment, which suggests that Hsp90α may be used as a marker for the evaluation of medical treatment in the above types of cancer.

In the embodiments of the present disclosure, the plasma Hsp90α concentrations in gastric cancer, pancreatic cancer, ovarian cancer, lymphoma, esophageal cancer, melanoma, kidney cancer, uterine cancer, nasopharyngeal cancer, osteosarcoma, bladder cancer, and prostate cancer patients were detected, which were significantly higher than those of healthy people (having statistical significance). The average reference value of plasma Hsp90α concentration in the above types of cancer was also provided. This suggests that tumor biomarker Hsp90α may be used in the diagnosis of gastric cancer, pancreatic cancer, ovarian cancer, lymphoma, esophageal cancer, melanoma, kidney cancer, uterine cancer, nasopharyngeal cancer, osteosarcoma, bladder cancer, prostate cancer, and other types of cancer.

### Brief Description of the Drawings

FIG. 1 depicts the ROC curve of Hsp90α detection results (based on the data from all the subjects whose Hsp90α concentration was detected).
FIG. 2 depicts the ROC curve of Hsp90α detection results, AUC 95% CI (0.788, 0.829).
FIG. 3 depicts the ROC curve of Hsp90α detection results, AUC 95%CI (0.811, 0.854).
FIG. 4 depicts the ROC curve of Hsp90α detection results, AUC 95%CI (0.804, 0.850).
FIG. 5 depicts the ROC curve of Hsp90α detection results, AUC 95%CI (0.731, 0.777).
FIG. 6 depicts the ROC curve of tumor enlargement determined by the slope.
FIG. 7 depicts the ROC curve of PD based on the percentage of change.
FIG. 8 depicts the ROC curve of PD based on the change of value.
FIG. 9 depicts the comparison of the samples from EDTA-K2 blood collection tube and EDTA-K3 blood collection tube.
FIG. 10 depicts the ROC curve of liver cancer patients vs. healthy people.
FIG. 11 depicts the ROC curve of liver cancer patients vs. non-cancerous liver disease patients.
FIG. 12 depicts the ROC curve of colorectal cancer patients vs. healthy people and non-cancerous colorectal disease patients.
FIG. 13 depicts the ROC curve of breast cancer patients vs. healthy people.
FIG. 14 depicts the ROC curve of breast cancer patients vs. non-cancerous breast disease patients.
FIG. 15 depicts the results of detected plasma Hsp90α concentrations of all the patients suffering from various types of cancer.

### Detailed Description of the Invention

The term "blood sample" refers to a sample obtained from the blood of a subject. In consideration of the interference of intracellular Hsp90α to the detection results, the blood sample is preferably a plasma sample or a serum sample, or a diluted plasma or serum sample. A skilled artisan would understand that due to the difference in the components of plasma and serum, the concentration of Hsp90α detected from a plasma sample and from a serum sample might be slightly different. For the convenience of quantification and comparison, a plasma sample is used in the present invention.

The terms "sensitivity", "specificity", and "accuracy" as used herein have the same meanings as commonly recognized in the field of medical statistics.

"Specificity" means the ratio of the samples which are determined by the kit to be "negative" to the samples which have been determined to be "non-cancerous" by pathological diagnosis.

"Sensitivity" means the ratio of the samples which are determined by the kit to be "positive" to the samples which have been determined to be "cancerous" by pathological diagnosis.

"Accuracy" means the ratio of the sum of true positive samples and true negative samples to the total samples, which reflects the extent to which the results detected by the kit are coincident with the exact conditions of the subjects (i.e. having cancer or not).

The terms "cut-off value", "dividing value", "reference value" and "threshold" as used herein may be used interchangeably, which refer to the standard used to judge the detection results, also known as critical value. Samples having a detection result higher than the cut-off value are considered to be "positive", and samples having a detection result lower than the cut-off value are considered to be "negative".

The term "clinical benefit" as used herein means that the disease remains stable or a relief is achieved after a treatment. CR, PR and SD have the same meanings as the terms in Response Evaluation Criteria in Solid Tumors (RECIST). Namely, CR means complete response, PR means partial response, SD means stable disease. The three indicators, CR, PR, and SD, are used to describe the people obtaining a clinical benefit. In contrary, PD means progressive disease.

The term "non-cancerous disease" means a benign disease which is not cancer as confirmed by pathological diagnosis. For example, in the study of lung cancer, it is used to describe patients who are diagnosed to have pneumonia, pulmonary tuberculosis, or other benign lung diseases which are not lung cancer. In the study of liver cancer, it is used to describe patients who are diagnosed to have hepatitis, hepatocirrhosis, or other liver diseases which are not liver cancer. In the study of colorectal cancer, it is used to describe patients who are diagnosed to have inflammation, colorectal polyps or other colorectal diseases which are not colorectal cancer. In the study of breast cancer, it is used to describe patients who are diagnosed to have mastitis, cyclomastopathy, or other breast diseases which are not breast cancer.

Meanings of a number of abbreviations and statistical terms as used herein are shown as below:

| | |
|---|---|
| CEA | Carcino-Embryonic Antigen |
| DOR | Diagnostic Accuracy Odds Ratio |
| CYFRA21-1 | Cytokerantin-19 Fragment 21-1 |
| AFP | α-Fetoprotein |
| CA125 | Cancer Antigen 125 |
| CA19-9 | Cancer Antigen 19-9 |
| CA15-3 | Cancer Antigen 15-3 |
| PSA | Prostate Specific Antigen |
| CA72-4 | Cancer Antigen 72-4 |
| SD | Standard Deviation |
| Hsp90α | Heat shock protein 90α |
| Mean | Mean |
| Md | Median |
| Min | Minimum |
| Max | Maximum |
| CI | Confidence Interval |
| Q1 | First Quartile |
| Q3 | Third Quartile |
| ROC | Receiver Operator Characteristic |
| 95%CI | 95% Confidence interval |

### Examples

### Example 1

### Research purpose

The clinical performance and the scope of clinical utility of the quantitative detection kit for human plasma heat shock protein 90α (Hsp90α) were evaluated by the following three tests.
1. To test the sensitivity, specificity, and accuracy of the quantitative detection kit for Hsp90α, concentration of plasma Hsp90α in healthy people, lung cancer patients, patients with non-cancerous lung diseases, and patients with other malignant tumors were detected. Suitable cut-off values were derived from the receiver-operating characteristic (ROC) curves for positive determination.
2. The concentration of plasma Hsp90α in lung cancer patients was dynamically monitored during the process of treatment, and the relevance between plasma Hsp90α concentration and the condition of patient was tested to evaluate its efficiency for efficacy monitoring.
3. Detection results of carcino-embryonic antigen (CEA) and cytokeratin 19 fragment antigen 21-1 (CYFRA21-1) were separately compared with those of Hsp90α, to compare the clinical performance of these tumor biomarkers, which was another purpose of the clinical trial.

### 1.1 Resource of samples

The selected subjects included inpatients and outpatients. No less than 1100 plasma samples in the non-dynamic monitoring group and plasma samples from no less than 150 patients in the dynamic monitoring group were studied. The ratio of high-value samples to low-value samples met the requirement of statistics.

### 1.1.1 Selection of non-dynamic monitoring subjects

Subjects included: case group and control group, no sex limitation.
(1) Case group: lung cancer patients who were newly diagnosed as tumor bearing by pathological method, including different types and different stages of lung cancer.
(2) Control group: people having no lung cancer as confirmed by biochemical, imaging, or pathological method. The control group shall include: healthy people having no apparent disease symptoms, people having no lung cancer but having other lung disease (e.g. pulmonary tuberculosis, pneumonia), people having benign lung tumor, and people having other malignant cancer.

Healthy people: humans having no known benign or malignant disease, having normal appearance, and having no visible disease symptom.

Non-cancerous lung disease patients: patients who were confirmed by biochemical, imaging, or pathological method to suffer from a lung disease which is not lung cancer.

### 1.1.2 Selection of dynamic monitoring subjects

At least 150 lung cancer patients who received standard medical treatment or surgical treatment were monitored continuously, so as to determine the relevance of the changes of plasma Hsp90α concentration to the changes of patient condition. Plasma samples were collected at the following time points:
(1) 12 lung cancer patients who received a surgical treatment were monitored at multiple time points so as to monitor the metabolism of Hsp90α in plasma. Plasma samples were collected before surgery and 1, 3, 7, 14, 21 days after surgery, respectively.
(2) The concentration of plasma Hsp90α of no less than 38 patients who received a surgical treatment was dynamically monitored: a plasma sample was collected within 3 days before surgery; another plasma sample was collected 3-7 days after surgery; and a plasma sample was collected within 3 days around the evaluation of clinical efficacy (i.e. the date of imaging examination). As such, at least a total of 3 samples were collected.
(3) The concentration of plasma Hsp90α of no less than 100 lung cancer patients who received a medical treatment was dynamically monitored: a plasma sample was first collected before the treatment. After the start of the treatment, a sample was collected at the end of each treatment cycle until all the four treatment cycles were finished. If a patient received a routine evaluation of clinical efficacy (imaging examination) after a treatment cycle was completed, a plasma sample should be collected within 3 days around the date of clinical evaluation (the date of imaging examination).

Previous samples that met the dynamic monitoring protocol could also be used in the study.

### 1.1.3 Sample exclusion criteria

(1) Samples from lung cancer patients who are receiving an ongoing radiotherapy to the tumor focus in lung;
(2) In dynamic monitoring process, samples from patients who are pregnant or in breast-feeding or from patients who are fertile but do not take contraceptive measures during the trial;
(3) In dynamic monitoring process, samples from patients in which the concentration of plasma Hsp90α before the treatment is lower than the cut-off value;
(4) Samples from patients suffering from other types of cancer.

### 1.2 Quantitative detection kit

### Product to be studied:

Quantitative detection kit for human plasma Hsp90α (ELISA)
Specification: 96 tests/Kit. Manufacturer: Yantai Protgen Biotechnology Development Co., Ltd.
Lot number: 20111018, 20120109, 20120505. Shelf life: 6 months. Storage condition: 2-8 °C.

### Products to be compared:

Because no similar products had been registered and marketed in China or abroad, pathological diagnosis and clinical diagnosis of lung cancer were used as the standard for evaluation.

In the study, carcinoembryonic antigen (CEA) and cytokeratin 19 fragment antigen 21-1 (CYFRA21-1) were used as reference to investigate the clinical performance of the kit.

### Example 2

### 2. Summary of the results

### 2.1 Non-dynamic monitoring group

2036 cases (lung cancer group: 1,046 cases, other malignant tumors group: 37 cases, non-cancerous lung diseases group: 344 cases, lung benign tumors group: 17 cases, healthy people group: 592 cases) were recruited in non-dynamic monitoring group. All 2036 cases met the recruitment standard. No subject was eliminated. For details, see Table 8.1.1.1 and Table 8.1.1.2. Case distribution from various centers was summarized in Table 8.1.1.3.

### The evaluation of diagnostic performance of Hsp90α

To evaluate the performance of Hsp90α in the diagnosis of lung cancer and non-cancerous diseases, patients suffering from other types of malignant tumors were excluded. 1999 cases were recruited in the study, among which 1046 cases were lung cancer patients and 953 cases were non-cancerous disease patients. The demographic information of the two groups and summary of the diagnostic results of lung cancer patients were shown in Table 8.1.1.1.1.1 and Table 8.1.1.1.1.2.

The difference between the plasma Hsp90α detection results of the two groups was statistically significant (P<0.0001), as shown in Table 8.1.1.1.2.1.

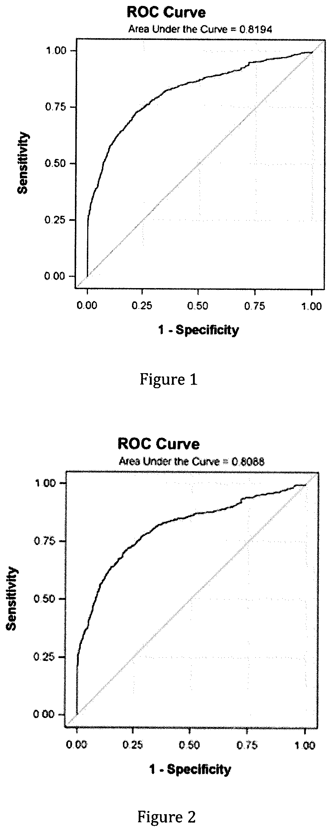

The area under the ROC curve of Hsp90α in lung cancer diagnosis was 0.8149. The optimal cut-off value determined by the maximum youden index was >56.33 ng/ml, corresponding to a sensitivity of 72.18% (95% confidence interval: 69.46% ∼ 74.90%) and a specificity of 78.7% (95% confidence interval: 76.10% ∼ 81.30%). The corresponding sensitivity and other diagnostic performance indicators corresponding to specificity 80%, 85%, 90%, and 95% were also described. See Figure 1 and Table 8.1.1.1.2.2 and Table 8.1.1.1.2.3.

In addition, the ROC curves of the diagnostic performance of Hsp90α for pulmonary adenocarcinoma, lung squamous cell carcinoma, and small cell lung cancer were similar with the ROC curve for all types of lung cancer. When comparing lung cancer group with non-cancerous lung disease group, the difference of plasma Hsp90α detection results between these two groups was statistically significant (P < 0.0001).

### Diagnostic performance of Hsp90α and CEA, and the evaluation of combination diagnosis of Hsp90α and CEA

To evaluate the diagnostic performance of Hsp90α and CEA, samples from subjects who had both Hsp90α and CEA detection results were subject to the evaluation. A total of 1056 cases were analyzed, including 713 cases of lung cancer, and 343 cases of non-cancerous lung diseases. Hsp90α cut-off value was set to be >56.33 and CEA cut-off value was set to be >5. If a positive result was obtained from either Hsp90α detection or CEA detection, the subject was determined to be positive. The combined use of Hsp90α and CEA showed further improved diagnostic sensitivity.

### Diagnostic performance of Hsp90α and CYFRA21-1, and the evaluation of combination diagnosis of Hsp90α and CYFRA21-1

To evaluate the diagnostic performance of Hsp90α and CYFRA21-1, samples from subjects who had both Hsp90α and CYFRA21-1 detection results were subject to the evaluation. A total of 910 cases were analyzed, including 660 cases of lung cancer and 250 cases of non-cancerous diseases. Hsp90α cut-off value was set to be >56.33 and CEA cut-off value was set to be >5. If a positive result was obtained from either Hsp90α detection or CYFRA21-1 detection, the subject was determined to be positive. The combined use of Hsp90α and CYFRA21-1 showed further improved diagnostic sensitivity.

### 2.2 Surgical group

106 cases were recruited in this evaluation, among which 79 were valid cases who had a plasma Hsp90α concentration not less than the cut-off value. The difference between the plasma Hsp90α concentrations taken before surgery and after surgery was statistically significant (P=0.0062<0.01). See Table 8.2.2 for details.

### 2.3 Dynamic monitoring group (patients receiving a medical treatment)

202 lung cancer patients were recruited in the evaluation, and the number of valid cases was 169. The demographic information and other basic information were listed in Table 8.3.1.2.

### Interval evaluation

A total of 284 valid statistical units were obtained, which included 69 cases in tumor shrinkage group, 161 cases in stable tumor size group, and 54 cases in tumor progression group. The median values of the changes of plasma Hsp90α concentration with reference to the baseline level as detected in the second treatment cycle in these three groups were -48.64 ng/ml, -8.51 ng/ml, 74.66 ng/ml, respectively. The differences were statistically significant (P <0.0001) by rank sum testing. In addition, differences between detection values obtained before and after the treatment within each group were statistically significant, and the median of percentage of change was -41.09%, -9.46%, and 65.26%, respectively.

The area under the ROC curve of Hsp90α for tumor enlargement diagnosis was 0.7719. It was determined that the best cut-off point was a change of >34.17% with reference to the baseline according to the youden index, and the corresponding sensitivity was 70.37% (95% confidence interval: 58.19%, 82.55 %), the corresponding specificity was 80% (95% confidence interval: 74.83%, 85.17%), as shown in Table 8.3.2.3.2.

### Evaluation by case

A total of 275 valid cases were obtained, which included 95 cases in partial response (PR) group, 118 cases in stable disease (SD) group, and 62 cases of progressive disease (PD) group. The medians of changes of Hsp90α concentration in comparison with baseline level in these groups were -90.98 ng/ml, -32.91 ng/ml, 80.32 ng/ml, respectively, which were statistically significant (P <0.0001) as tested by rank sum. Differences between Hsp90α detection values obtained before and after treatment within each group were all statistically significant, and the medians of percentages of change were -48.16%, -22.23%, 71.40%, respectively.

The area under the ROC curve of Hsp90α for PD diagnosis was 0.8406. It was determined that the best cut-off point was a change of >34.17% as compared with baseline according to the youden index, and the corresponding sensitivity was 72.58% (95% confidence interval: 61.48%, 83.68%), the corresponding specificity was 85.45% (95% confidence interval: 80.71%, 90.18%), as shown in Table 8.3.3.2.2.

### Example 3

### 3 Statistical results

### 3.1 Non-dynamic monitoring group

**Table 8.1.1.1 Cases recruited and dataset for analysis**

| **Cases** | **Results (Percentage)** |
|---|---|
| **All cases** | 2036 (100%) |
| **Excluded cases** | 0 (0.00%) |
| **Valid cases** | 2036 (100%) |
| **Lung cancer patients** | 1046 (51.38%) |
| **Other malignant cancer patients** | 37 (1.82%) |
| **Non-cancerous lung disease patients** | 344 (16.90%) |
| **Benign lung tumor patients** | 17 (0.83%) |
| **Healthy people** | 592 (29.08%) |

**Table 8.1.1.2 Case distribution in non-dynamic monitoring group**

| **Clinical Centers** | **Cases recruited** | **Valid cases** |
|---|---|---|
| **1** | 141 | 141 |
| **2** | 857 | 857 |
| **3** | 137 | 137 |
| **4** | 15 | 15 |
| **5** | 118 | 118 |
| **7** | 60 | 60 |
| **8** | 480 | 480 |
| **9** | 228 | 228 |
| **Sum** | 2036 | 2036 |

### 3.1.1 Lung cancer group and non-cancerous group

### 3.1.1.1 Evaluation of the diagnostic performance of Hsp90α

### 3.1.1.1.1 Characteristics of subjects

**Table 8.1.1.1.1.1 Demographic information of all subjects**

| **Index** | **Lung cancer group** | **Non-cancerous group** | **Total** |
|---|---|---|---|
| **Age (years)** | | | |
| **N (Missing)** | 1043 (3) | 869 (84) | 1912 (87) |
| **Mean (SD)** | 59.76(10.79) | 47.74(17.93) | 54.30(15.67) |
| **Min, Max** | 24,100 | 1098 | 10,100 |
| **Md (Q3-Q1)** | 60.00(13.00) | 46.00(24.00) | 55.00(22.00) |

| **Sex** | | | |
|---|---|---|---|
| **Male** | 711(68.23%) | 468(49.95%) | 1179(59.58%) |
| **Female** | 331(31.77%) | 469(50.05%) | 800(40.42%) |
| **In sum** | 1042 | 937 | 1979 |
| **Missing** | 4 | 16 | 20 |

**Table 8.1.1.1.1.2 Diagnostic results of lung cancer patients**

| **Index** | **Results** |
|---|---|
| **Types of lung cancer** | |
| **Adenocarcinoma** | 537(52.54%) |
| **Squamous cell carcinoma** | 218(21.33%) |
| **Small cell carcinoma** | 136(13.31%) |
| **Large cell carcinoma** | 4(0.39%) |
| **Undefined** | 25(2.45%) |
| **Others** | 30(2.94%) |
| **NK** | 72(7.05%) |
| **Total** | 1022 |
| **Missing** | 24 |

| **Small cell carcinoma staging** | |
|---|---|
| **Diffused** | 10(11.49%) |
| **Local** | 18(20.69%) |
| **NK** | 59(67.82%) |
| **Total** | 87 |
| **Missing** | 959 |

| **Differentiation of lung cancer** | |
|---|---|
| **Poorly-differentiated** | 122(17.43%) |
| **Middle- to low- differentiated** | 16(2.29%) |
| **Moderate-differentiated** | 60(8.57%) |
| **Well-differentiated** | 9(1.29%) |
| **NK** | 493(70.43%) |
| **Total** | 700 |
| **Missing** | 346 |

### 3.1.1.1.2 Evaluation of diagnostic performance of Hsp90α (lung cancer group and non-cancerous group)

**Table 8.1.1.1.2.1 Detection results of plasma Hsp90α in lung cancer group and non-cancerous group**

| **Item** | **Lung cancer group** | **Non-cancerous group** | **Test statistic** | **p-value** |
|---|---|---|---|---|
| **Hsp90α (ng/ml)** | | | 15.08(t test) | <0.0001 |
| **N(missing)** | 1046(0) | 953(0) | | |
| **Mean(SD)** | 220.46(351.55) | 48.00(34.82) | | |
| **Min, Max** | 7.07, 7400 | 0.95, 371.37 | | |
| **Md(Q3-Q1)** | 95.54(199.97) | 38.07(24.41) | | |

Figure 1 shows the ROC curve of plasma Hsp90α detection results (based on all subjects)

**Table 8.1.1.1.2.2 Hsp90α diagnosis in lung cancer based on different cut-off values**

| **Diagnosis by Hsp90α** | **Diagnosis** | | |
|---|---|---|---|
| | Positive | Negative | Total |
| **>56.33** | | | |
| **Positive** | 755(72.18%) | 203(21.30%) | 958(47.92%) |
| **Negative** | 291(27.82%) | 750(78.70%) | 1041(52.08%) |
| **Total** | 1046 | 953 | 1999 |

| **>58.17** | | | |
|---|---|---|---|
| **Positive** | 735(70.27%) | 191(20.04%) | 926(46.32%) |
| **Negative** | 311(29.74%) | 762(79.96%) | 1073(53.68%) |
| **Total** | 1046 | 953 | 1999 |

| **>67.47** | | | |
|---|---|---|---|
| **Positive** | 674(64.44%) | 143(15.01%) | 817(40.87%) |
| **Negative** | 372(35.56%) | 810(84.99%) | 1182(59.13%) |
| **Total** | 1046 | 953 | 1999 |

| **>80.99** | | | |
|---|---|---|---|
| **Positive** | 600(57.36%) | 95(9.97%) | 695(34.77%) |
| **Negative** | 446(42.64%) | 858(90.03%) | 1304(65.23%) |
| **Total** | 1046 | 953 | 1999 |

| **>117.36** | | | |
|---|---|---|---|
| **Positive** | 444(42.45%) | 48(5.04%) | 492(24.61%) |
| **Negative** | 602(57.55%) | 905(94.96%) | 1507(75.39%) |
| **Total** | 1046 | 953 | 1999 |

**Table 8.1.1.1.2.3 Hsp90α diagnosis based on different cut-off values**

| **Index** | **TP** | **TN** | **FP** | **FN** | **Sensitivity** | | **Specificity** | | **Accuracy(%)** | **PPV(%)** | **NPV(%)** | **LR+** | **LR-** | **DOR** |
|---|---|---|---|---|---|---|---|---|---|---|---|---|---|---|
| | | | | | % | %95CI | % | %95CI | | | | | | |
| Hsp90α (ng/ml) | | | | | | | | | | | | | | |
| >56.33 | 755 | 750 | 203 | 291 | 72.18 | 69.46,74.90 | 78.7 | 76.10,81.30 | 75.29 | 78.81 | 72.05 | 3.39 | 0.35 | 9.69 |
| >58.17 | 735 | 762 | 191 | 311 | 70.27 | 67.50,73.04 | 79.96 | 77.42,82.50 | 74.89 | 79.37 | 71.02 | 3.51 | 0.37 | 9.49 |
| >67.47 | 674 | 810 | 143 | 372 | 64.44 | 61.53,6734 | 84.99 | 82.73,87.26 | 74.24 | 82.5 | 68.53 | 4.29 | 0.42 | 10.21 |
| >80.99 | 600 | 858 | 95 | 446 | 57.36 | 5436,6036 | 90.03 | 88.13,91.93 | 72.94 | 8633 | 65.8 | 5.75 | 0.47 | 12.23 |
| >11736 | 444 | 905 | 48 | 602 | 42.45 | 39.45,45.44 | 94.96 | 93.57,96.35 | 67.48 | 90.24 | 60.05 | 8.42 | 0.61 | 13.8 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

**Table 8.1.1.1.2.4 The difference of plasma Hsp90α concentration between early (I-II) stage lung cancer and late (III-IV) stage lung cancer**

| **Index** | **I, II stages of lung cancer** | **III, IV stages of lung cancer** | **Test statistic** | **P value** |
|---|---|---|---|---|
| **Hsp90α (ng/ml)** | | | -4.19(Rank sum test) | <0.0001 |
| **N(missing)** | 93(0) | 720(0) | | |
| **Mean(SD)** | 111.50(130.82) | 251.38(300.69) | | |
| **Min, Max** | 7.07,1043.7 | 11.18,2398 | | |
| **Md(Q3-Q1)** | 82.24(55.65) | 117.33(311.16) | | |

### 3.1.1.1.3 Evaluation of the diagnostic performance of Hsp90α (adenocarcinoma group vs. non-cancerous group)

Figure 2 shows the ROC curve of Hsp90α in adenocarcinoma group vs. non-cancerous group, area under curve (AUC) (95%CI: 0.788, 0.829)

### 3.1.1.1.4 Evaluation of the diagnostic performance of Hsp90α (squamous cell carcinoma group vs. non-cancerous group)

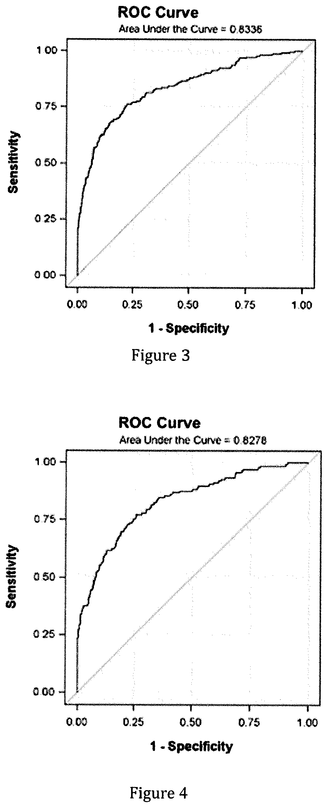

Figure 3 shows the ROC curve of Hsp90α in squamous cell carcinoma group vs. non-cancerous group, AUC (95%CI: 0.811, 0.854)

### 3.1.1.1.5 Evaluation of the diagnostic performance of Hsp90α (small cell carcinoma group vs. non-cancerous group)

Figure 4 shows the ROC curve of Hsp90α in small cell carcinoma group vs. non-cancerous group, AUC (95%CI: 0.804, 0.850)

### 3.1.1.1.6 Evaluation of the diagnostic performance of Hsp90α (lung cancer group vs. lung non-cancerous disease group)

**Table 8.1.1.1.6.1 Detection results of Hsp90α in lung cancer and lung non-cancerous disease group**

| **Index** | **Lung cancer** | **Non-cancerous group** | **Test statistic** | **P value** |
|---|---|---|---|---|
| **Hsp90α (ng/ml)** | | | -14.20(Rank sum test) | <0.0001 |
| **N(Missing)** | 1046(0) | 344(0) | | |
| **Mean(SD)** | 220.46(351.55) | 58.58(39.43) | | |
| **Min, Max** | 7.07,7400 | 11.61,315.57 | | |
| **Md(Q3-Q1)** | 95.54(199.97) | 48.48(31.85) | | |

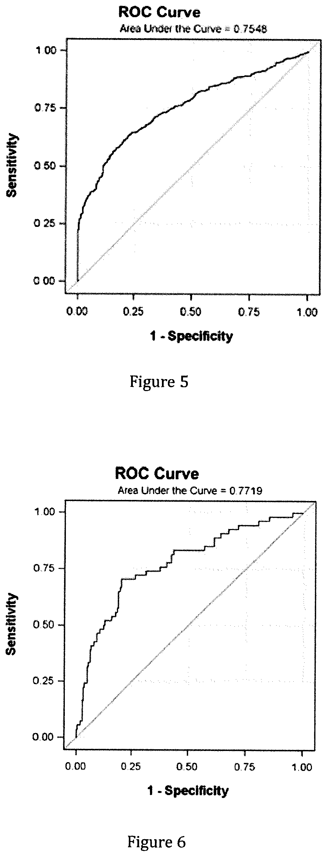

Figure 5 shows the ROC curve of Hsp90α in lung cancer group vs. lung non-cancerous disease group, AUC (95%CI: 0.731, 0.777)

**Table 8.1.1.1.6.28 Diagnosis results based on different cut-off vales**

| **Index** | **TP** | **TN** | **FP** | **FN** | **Sensitivity** | | **Specificity** | | **Accuracy** | **PPV** | **NPV** | **LR+** | **LR-** | **DOR** |
|---|---|---|---|---|---|---|---|---|---|---|---|---|---|---|
| | | | | | % | %95CI | % | %95CI | **(%)** | **(%)** | **(%)** | | | |
| Hsp90α (ng/ml) | | | | | | | | | | | | | | |
| >74.18 | 639 | 275 | 69 | 407 | 61.09 | 58.14,64.04 | 79.94 | 75.71,84.17 | 65.76 | 90.25 | 40.32 | 3.05 | 0.49 | 6.22 |
| >8339 | 586 | 292 | 52 | 460 | 56.02 | 53.01,59.03 | 84.88 | 81.10,88.67 | 63.17 | 91.85 | 38.83 | 3.71 | 0.52 | 7.13 |

### 3.1.1.2 Diagnostic performance of Hsp90α and CEA

### 3.1.1.2.1 Characteristics of subjects

**Table 8.1.1.2.1.1 Demographic information of all subjects**

| **Index** | **Lung cancer group** | **Non-cancerous group** | **Total** |
|---|---|---|---|
| **Age(years)** | | | |
| **N(Missing)** | 710(3) | 293(50) | 1003(53) |
| **Mean(SD)** | 60.21(10.62) | 47.63(20.41) | 56.53(15.29) |
| **Min, Max** | 29,100 | 12,98 | 12,100 |
| **Md(Q3-Q1)** | 60.00(14.00) | 46.00(34.00) | 58.00(19.00) |

| **Sex** | | | |
|---|---|---|---|
| **Male** | 493(69.44%) | 191(56.51) | 684(65.27%) |
| **Female** | 217(30.56%) | 147(43.49%) | 364(34.73%) |
| **Total** | 710 | 338 | 1048 |
| **Missing** | 3 | 5 | 8 |

**Table 8.1.1.2.1.2 Diagnosis results of lung cancer patients**

| **Index** | **Results** |
|---|---|
| **Types of lung cancer** | |
| **Adenocarcinoma** | 348(50.36%) |
| **Squamous cell carcinoma** | 131(18.96%) |
| **Small cell carcinoma** | 116(16.79%) |
| **Large cell carcinoma** | 1(0.14%) |
| **Undefined** | 20(2.89%) |
| **Others** | 16(2.32%) |
| **NK** | 59(8.54%) |
| **Total** | 691 |
| **Missing** | 22 |

| **Stages of small cell carcinoma** | |
|---|---|
| **Diffused** | 8(11.59%) |
| **Local** | 16(23.19%) |
| **NK** | 45(65.22%) |
| **Total** | 69 |
| **Missing** | 644 |

| **Differentiation degree of lung cancer** | |
|---|---|
| **Poorly-differentiated** | 90(22.22%) |
| **Middle- to low- differentiated** | 3(0.74%) |
| **Moderate-differentiated** | 9(2.22%) |
| **Well-differentiated** | 1(0.25%) |
| **NK** | 302(74.57%) |
| **Total** | 405 |
| **Missing** | 308 |

### 3.1.1.2.2 Evaluation of diagnostic performance of Hsp90α and CEA (lung cancer group and non-cancerous group)

**Table 8.1.1.2.2.1 Detection results of Hsp90α and CEA in lung cancer group and non-cancerous group**

| **Index** | **Lung cancer group** | **Non-cancerous group** | **Test statistic** | **P value** |
|---|---|---|---|---|
| **Hsp90α (ng/ml)** | | | 8.71(t test) | <0.0001 |
| **N(Missing)** | 713(0) | 343.0 | | |
| **Mean(SD)** | 229.21(383.65) | 48.49(32.80) | | |
| **Min, Max** | 19.77,7400 | 0.95,315.57 | | |
| **Md(Q3-Q1)** | 96.44(249.27) | 40.20(24.52) | | |
| **CEA (ng/ml)** | | | 5.24(t test) | <0.0001 |
| **N(Missing)** | 713(0) | 343(0) | | |
| **Mean(SD)** | 40.22(119.66) | 4.77(53.92) | | |
| **Min, Max** | 0.05,1000 | 0.03,1000 | | |
| **Md(Q3-Q1)** | 4.80(17.05) | 1.48(1.67) | | |

### 3.1.1.2.2.1 Evaluation of diagnostic performance of the combined use of Hsp90α and CEA (Lung cancer group and non-cancerous group)

**Table 8.1.1.2.2.3.1 Fourfold table of combination diagnosis of Hsp90α and CEA (determined to be positive if either Hsp90α or CEA is positive)**

| **Combination diagnosis** | **Clinical diagnosis** | | |
|---|---|---|---|
| | Positive | Negative | Total |
| **Hsp90α+CEA*** | | | |
| **Positive** | 600(84.15%) | 83(24.20%) | 683(64.68%) |
| **Negative** | 113(15.85%) | 260(75.80%) | 373(35.32%) |
| **Total** | 713 | 343 | 1056 |

| **Hsp90α+CEA**** | | | |
|---|---|---|---|
| **Positive** | 603(84.57%) | 86(25.07%) | 689(65.25%) |
| **Negative** | 110(15.43%) | 257(74.93%) | 367(34.75%) |
| **Total** | 713 | 343 | 1056 |

| | | | |
|---|---|---|---|
| Note: * Cut-off value of Hsp90α was 56.33, and cut-off value of CEA was 5. **Cut-off value of Hsp90α was 56.33, and cut-off value of CEA was 4.74. | | | |

**Table 8.1.1.2.2.3.2 Results of combination diagnosis of Hsp90α and CEA**

| Index | TP | TN | FP | FN | **Sensitivity** | | **Specificity** | | Accuracy | PPV (%) | NPV (%) | LR+ | LR- | DOR |
|---|---|---|---|---|---|---|---|---|---|---|---|---|---|---|
| | | | | | % | %95CI | % | %95CI | | | | | | |
| Combination diagnosis | 600 | 260 | 83 | 113 | 84.15 | 81.47,86.83 | 75.8 | 71.27,80.33 | 81.44 | 87.85 | 69.71 | 3.48 | 0.21 | 16.57 |
| | 603 | 257 | 86 | 110 | 84.57 | 81.92,87.22 | 74.93 | 70.34,79.51 | 81.44 | 87.52 | 70.03 | 3.37 | 0.21 | 16.05 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

### 3.1.1.3 Diagnostic performance of Hsp90α and CYFRA21-1

### 3.1.1.3.1 Characteristics of subjects

**Table 8.1.1.3.1.1 The demographic information of all subjects**

| **Index** | **Lung cancer group** | **Non-cancerous group** | **Total** |
|---|---|---|---|
| **Age (year)** | | | |
| **N (Missing)** | 659(1) | 199(51) | 858(52) |
| **Mean (SD)** | 60.25(10.35) | 43.65(18.83) | 56.40(14.60) |
| **Min, Max** | 30,89 | 12,98 | 12,98 |
| **Md(Q3-Q1)** | 60.00(14.00) | 42.00(32.00) | 58.00(19.00) |

| **Sex** | | | |
|---|---|---|---|
| **Male** | 461(70.06%) | 141(57.32%) | 602(66.59%) |
| **Female** | 197(29.94%) | 105(42.68%) | 302(33.41%) |
| **Total** | 658 | 246 | 904 |
| **Missing** | 2 | 4 | 6 |

**Table 8.1.1.3.1.2 Diagnosis results of lung cancer patients**

| **Index** | **Results** |
|---|---|
| **Types of lung cancer** | |
| **Adenocarcinoma** | 317(49.53%) |
| **Squamous cell carcinoma** | 125(19.53%) |
| **Small cell carcinoma** | 110(17.19%) |
| **Large cell carcinoma** | 1(0.16%) |
| **Undefined** | 20(3.13%) |
| **Others** | 13(2.03%) |
| **NK** | 54(8.44%) |
| **Total** | 640 |
| **Missing** | 20 |

| **Stages of small cell carcinoma** | |
|---|---|
| **Diffused** | 8(12.50%) |
| **Local** | 15(23.44%) |
| **NK** | 41(64.06%) |
| **Total** | 64 |
| **Missing** | 596 |

| **Differentiation of lung cancer** | |
|---|---|
| **Poorly-differentiated** | 88(22.56%) |
| **Middle- to low-differentiated** | 2(0.51%) |
| **Morderate-differentiated** | 8(2.05%) |
| **Well-differentiated** | 1(0.26%) |
| **NK** | 291(74.62%) |
| **Total** | 390 |
| **Missing** | 270 |

### 3.1.1.3.2 Evaluation of diagnostic performance of Hsp90α and CYFRA21-1 (Lung cancer group and non-cancerous group)

**Table 8.1.1.3.2.1.1 Detection results of lung cancer group and non-cancerous group**

| **Index** | **Lung cancer group** | **Non-cancerous group** | **Test statistic** | **P value** |
|---|---|---|---|---|
| **Hsp90α (ng/ml)** | | | 7.59(t test) | <0.0001 |
| **N (Missing)** | 660(0) | 250(0) | | |
| **Mean (SD)** | 238.59(396.14) | 48.19(35.30) | | |
| **Min, Max** | 19.77,7400 | 0.95,315.57 | | |
| **Md (Q3-Q1)** | 98.18(280.04) | 38.40(23.91) | | |
| **CEA (ng/ml)** | | | 4.75(t test) | <0.0001 |
| **N Missing)** | 660(0) | 250(0) | | |
| **Mean (SD)** | 9.82(25.26) | 2.17(5.09) | | |
| **Min, Max** | 0.26,266.3 | 0.23,74.44 | | |
| **Md (Q3-Q1)** | 3.36(5.39) | 1.59(1.11) | | |

### 3.1.1.3.2.1 Evaluation of the diagnostic performance of the combination use of Hsp90α and CYFRA21-1 (lung cancer group and non-cancerous group)

**Table 8.1.1.3.2.3.1 Fourfold table for the combination diagnosis of Hsp90α and CYFRA21-1 (the result was determined to be positive if either Hsp90α or CYFRA21-1 was positive)**

| **Combination diagnosis** | **Clinical diagnosis** | | |
|---|---|---|---|
| | Positive | Negative | Total |
| **Hsp90α+CYFRA21-1 *** | | | |
| **Positive** | 532(80.61%) | 51(20.40%) | 583(64.07%) |
| **Negative** | 128(19.39%) | 199(79.60%) | 327(35.93%) |
| **Total** | 660 | 250 | 910 |

| **Hsp90α+CYFRA21-1 **** | | | |
|---|---|---|---|
| **Positive** | 574(86.97%) | 62(24.80%) | 636(69.89%) |
| **Negative** | 86(13.03%) | 188(75.20%) | 274(30.11%) |
| **Total** | 660 | 250 | 910 |

| | | | |
|---|---|---|---|
| Note: * Cut-off value of Hsp90α was 56.33, and cut-off value of CYFRA21-1 was 5. **Cut-off value of Hsp90α was 56.33, and cut-off value of CYFRA21-1 was 3.1. | | | |

**Table 8.1.1.3.2.3.2 Results of combination diagnosis of Hsp90α and CYFRA21-1**

| **Index** | **TP** | **TN** | **FP** | **FN** | **Sensitivity** | | **Specificity** | | **Accuracy** | **PPV (%)** | **NPV (%)** | **LR+** | **LR-** | **DOR** |
|---|---|---|---|---|---|---|---|---|---|---|---|---|---|---|
| | | | | | % | %95CI | % | %95CI | | | | | | |
| combination diagnosis | 532 | 199 | 51 | 128 | 80.61 | 77.59,83.62 | 79.6 | 74.60,84.60 | 8033 | 91.25 | 60.86 | 3.95 | 0.24 | 16.46 |
| | 574 | 188 | 62 | 86 | 86.97 | 84.40,89.54 | 75.2 | 69.85,80.55 | 83.74 | 90.25 | 68.81 | 3.51 | 0.17 | 20.65 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

### 3.2 Surgical group

**Table 8.2.1 Distribution of surgical patients in different centers**

| **Centers** | **Selected subjects** | **Valid subjects** |
|---|---|---|
| **3** | 96 | 76 |
| **5** | 13 | 3 |
| **Total** | 109 | 79 |

**Table 8.2.2 Changes of plasma Hsp90α concentrations detected before and after operation**

| **Index** | **Results** |
|---|---|
| **Before operation** | |
| **N (Missing)** | 79(0) |
| **Mean (SD)** | 134.33(97.40) |
| **Min, Max** | 56.39,597.98 |
| **Md (Q3-Q1)** | 96.97(81.84) |

| **After-operation** | |
|---|---|
| **N (Missing)** | 79(0) |
| **Mean (SD)** | 99.81(49.72) |
| **Min, Max** | 19.3,328.23 |
| **Md (Q3-Q1)** | 95.40(56.95) |

| **Before operation vs. after operation** | |
|---|---|
| **N (Missing)** | 79(0) |
| **Mean (SD)** | 34.52(109.04) |
| **Min, Max** | -248.92,472.15 |
| **Md (Q3-Q1)** | 20.79(77.44) |
| **T test (P value)** | 2.81(0.0062) |

### 3.3 Dynamic monitoring of medical treatment group

### 3.3.1 Characteristics of subjects

**Table 8.3.1.1 The demographic information of all subjects**

| **Index** | **Results** |
|---|---|
| **Age (years)** | |
| **N (Missing)** | 166(3) |
| **Mean (SD)** | 54.57(10.53) |
| **Min, Max** | 26,78 |
| **Md (Q3-Q1)** | 56.00(14.00) |

| **Sex** | |
|---|---|
| **Male** | 113(67.26%) |
| **Female** | 55(32.74%) |
| **Total** | 168 |
| **Missing** | 1 |

| **Types of lung cancer** | |
|---|---|
| **Adenocarcinoma** | 101(59.76%) |
| **Squamous cell carcinoma** | 46(27.22%) |
| **Small cell carcinoma** | 11(6.51%) |
| **Large cell carcinoma** | 1(0.59%) |
| **Undefined** | 2(1.18%) |
| **Others** | 7(4.14%) |
| **NK** | 1(0.59%) |
| **Total** | 169 |

| **Stages of small cell lung cancer** | |
|---|---|
| **Diffused** | 2(40.00%) |
| **Local** | 3(60.00%) |
| **NK** | 0(0.00%) |
| **Total** | 5 |

### 3.3.2 Interval evaluation

**Table 8.3.2.1 Detection results of plasma Hsp90α concentration after each treatment cycle in different groups**

| **Index** | **Baseline** | **First cycle** | **Second cycle** |
|---|---|---|---|
| **Tumor shrinkage group** | | | |
| **N(Missing)** | 69(0) | 69(0) | 69(0) |
| **Mean(SD)** | 338.02(385.23) | 177.59(208.86) | 167.36(154.62) |
| **Min, Max** | 23.3,1527 | 25.07,1093.9 | 13.61,761.9 |
| **Md(Q3-Q1)** | 152.65(315.18) | 106.87(147.44) | 94.00(166.27) |
| **Tumor stable group** | | | |
| **N(Missing)** | 161(0) | 161(0) | 161(0) |
| **Mean(SD)** | 250.49(284.15) | 229.90(249.42) | 209.70(227.12) |
| **Min, Max** | 6.53,2217.1 | 10.71,1882.5 | 11.71,1782.9 |
| **Md(Q3-Q1)** | 153.17(246.57) | 154.34(227.54) | 137.99(190.90) |

| **Tumor enlarged group** | | | |
|---|---|---|---|
| **N(Missing)** | 54(0) | 54(0) | 54(0) |
| **Mean(SD)** | 227.23(352.02) | 248.72(299.59) | 378.83(557.28) |
| **Min, Max** | 18.6,1877.1 | 23.68,1433.4 | 24.4,3625 |
| **Md(Q3-Q1)** | 114.22(156.79) | 125.76(238.81) | 198.91(273.14) |

| **Tumor shrinkage group + tumor stable group** | | | |
|---|---|---|---|
| **N(Missing)** | 230(0) | 230(0) | 230(0) |
| **Mean(SD)** | 276.75(319.53) | 214.21(238.74) | 197.00(208.61) |
| **Min, Max** | 6.53,2217.1 | 10.71,1882.5 | 11.71,1782.9 |
| **Md(Q3-Q1)** | 152.91(263.59) | 134.79(210.91) | 129.07(194.61) |

| **Tumor enlarged group + tumor stable group** | | | |
|---|---|---|---|
| **N(Missing)** | 215(0) | 215(0) | 215(0) |
| **Mean(SD)** | 244.65(301.93) | 234.63(262.31) | 252.18(347.69) |

**Table8.3.2.2 Comparison of Hsp90α concentrations after each treatment cycle in different groups**

| **Index** | **Tumor shrinkage group** | **tumor stable group** | **Tumor enlarged group** | **Test statistic** | **P-value** |
|---|---|---|---|---|---|
| **Baseline** | | | | 3.62(Rank sum test) | 0.1636 |
| **N(Missing)** | 69(0) | 161(0) | 54(0) | | |
| **Mean(SD)** | 338.02(385.23) | 250.49(284.15) | 227.23(352.02) | | |
| **Min, Max** | 23.3,1527 | 6.53,2217.1 | 18.6,1877.1 | | |
| **Md(Q3-Q1)** | 152.65(315.18) | 153.17(246.57) | 114.22(156.79) | | |
| **The 2^{nd} treatment cycle** | | | | 11.79(Rank sum test) | 0.0027 |
| **N(Missing)** | 69(0) | 161(0) | 54(0) | | |
| **Mean(SD)** | 167.36(154.62) | 209.70(227.12) | 378.83(557.28) | | |
| **Min, Max** | 13.61,761.9 | 11.71,1782.9 | 24.4,3625 | | |
| **Md(Q3-Q1)** | 94.00(166.27) | 137.99(190.90) | 198.91(273.14) | | |
| **The 2^{nd} treatment cycle vs. baseline** | | | | 42.91 (Rank sum test) | <0.0001 |
| **N(Missing)** | 69(0) | 161(0) | 54(0) | | |
| **Mean(SD)** | -170.7(306.58) | -40.80(187.55) | 151.61(524.36) | | |
| **Min, Max** | -1024.68,399.77 | -875.62,645.21 | -854.79,3370.22 | | |
| **Md(Q3-Q1)** | -48.64(225.17) | -8.51(102.35) | 74.66(159.12) | | |
| **Rank sum testing (P)** | -811.5(<0.0001) | -1352(0.0220) | 427.50(<0.0001) | | |

| **The percentage of the 2^{nd} treatment cycle vs. baseline** | | | | | |
|---|---|---|---|---|---|
| **N(Missing)** | 69(0) | 161(0) | 54(0) | | |
| **Mean(SD)** | -24.61(53.07) | 21.97(148.88) | 174.62(422.28) | | |
| **Min, Max** | -90.51,227.56 | -92.25,1241.98 | -81.86,2656.07 | | |
| **Md(Q3-Q1)** | -41.09(59.22) | -9.46(75.72) | 65.26(157.56) | | |

Figure 6 shows the ROC curve for the diagnosis of tumor enlargement based on the slope.

**Table 8.3.2.3.1 Fourfold table for the determination of tumor enlargement according to the percentage of changes vs. the baseline**

| **Index** | **Evaluation** | | |
|---|---|---|---|
| | Tumor enlarged group | Tumor shrinkage group + Tumor stable group | Total |
| **>0** | | | |
| Tumor enlarged group | 40(74.07%) | 86(37.39%) | 126(44.37%) |
| Tumor shrinkage group + Tumor stable group | 14(25.93%) | 144(62.61%) | 158(55.63%) |
| **Total** | 54 | 230 | 284 |

| **>10** | | | |
|---|---|---|---|
| Tumor enlarged group | 39(72.22%) | 72(31.30%) | 111(39.08%) |
| Tumor shrinkage group + Tumor stable group | 15(27.78%) | 158(68.70%) | 173(60.92%) |
| **Total** | 54 | 230 | 284 |

| **>20** | | | |
|---|---|---|---|
| Tumor enlarged group | 38(70.37%) | 61(26.52%) | 99(34.86%) |
| Tumor shrinkage group + Tumor stable group | 16(29.63%) | 169(73.48%) | 185(65.14%) |
| **Total** | 54 | 230 | 284 |

| **>34.17** | | | |
|---|---|---|---|
| Tumor enlarged group | 38(70.37%) | 46(20.00%) | 84(29.58%) |
| Tumor shrinkage group + Tumor stable group | 16(29.63%) | 184(80.00%) | 200(70.42%) |
| **Total** | 54 | 230 | 284 |

| **>40** | | | |
|---|---|---|---|
| Tumor enlarged group | 33(61.11%) | 43(18.70%) | 76(26.76%) |
| Tumor shrinkage group + Tumor stable group | 21(38.89%) | 187(81.30%) | 208(73.24%) |
| **Total** | 54 | 230 | 284 |

**Table 8.3.2.3.2 Diagnosis of tumor enlargement based on the percentage of change vs. baseline at different cut-off values**

| percentage of changes of Hsp90α vs. baseline | TP | TN | FP | FN | % | %95CI | % | %95CI | Accuracy(%) | PPV(%) | NPV(%) | LR+ | LR- | DOR |
|---|---|---|---|---|---|---|---|---|---|---|---|---|---|---|
| Tumor enlarged group vs. Tumor shrinkage group + Tumor stable group | | | | | | | | | | | | | | |
| >0 | 40 | 144 | 86 | 14 | 74.07 | 62.39,85.76 | 62.61 | 56.36,68.86 | 64.79 | 31.75 | 91.14 | 1.98 | 0.41 | 4.83 |
| >10 | 39 | 158 | 72 | 15 | 72.22 | 60.28,84.17 | 68.7 | 62.70,74.69 | 69.37 | 35.14 | 91.33 | 2.31 | 0.4 | 5.78 |
| >20 | 38 | 169 | 61 | 16 | 70.37 | 58.19,82.55 | 73.48 | 67.77,79.18 | 72.89 | 38.38 | 91.35 | 2.65 | 0.4 | 6.63 |
| >34.17 | 38 | 184 | 46 | 16 | 70.37 | 58.19,82.55 | 80 | 74.83,85.17 | 78.17 | 45.24 | 92 | 3.52 | 0.37 | 9.51 |
| >40 | 33 | 187 | 43 | 21 | 61.11 | 48.11,74.11 | 81.3 | 76.27,86.34 | 77.46 | 43.42 | 89.9 | 3.27 | 0.48 | 6.81 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

### 3.3.3 Evaluation of subjects

**Table 8.3.3.1 Comparison of Hsp90α concentrations in different efficacy groups**

| **Index** | **PR** | **SD** | **PD** | **Test statistic** | **P-value** |
|---|---|---|---|---|---|
| **Baseline** | | | | 16.96(Rank sum test) | 0.0002 |
| **N(Missing)** | 95(0) | 118(0) | 62(0) | | |
| **Mean(SD)** | 401.55(422.99) | 313.87(292.21) | 216.07(368.54) | | |
| **Min, Max** | 56.68,1527 | 57.93,1527 | 18.598,2217.1 | | |
| **Md(Q3-Q1)** | 179.94(574.25) | 194.03(281.54) | 111.56(156.79) | | |
| **At the time of evaluation** | | | | 12.82(Rank sum test) | 0.0016 |
| **N(Missing)** | 95(0) | 118(0) | 62(0) | | |
| **Mean(SD)** | 178.90(171.81) | 237.27(294.39) | 367.67(525.70) | | |
| **Min, Max** | 13.61,944.15 | 11.71,2217.1 | 24.402,3625 | | |
| **Md(Q3-Q1)** | 121.91(179.25) | 154.43(199.76) | 198.91(276.86) | | |
| **At the time of evaluation-Baseline** | | | | 65.40(Rank sum test) | <0.0001 |
| **N(Missing)** | 95(0) | 118(0) | 62(0) | | |
| **Mean(SD)** | -222.6(317.98) | -76.60(325.54) | 151.60(502.96) | | |
| **Min, Max** | -1024.68,153.61 | -1010.94,1821.5 2 | -1288.99,3370.22 | | |
| **Md(Q3-Q1)** | -90.98(369.94) | -32.91(244.66) | 80.32(169.60) | | |
| **Rank sum test (P)** | -1814(<0.0001) | -1424(<0.0001) | 647.50(<0.0001) | | |

| **The percentage of change at the time of evaluation compared with baseline** | | | | | |
|---|---|---|---|---|---|
| **N(Missing)** | 95(0) | 118(0) | 62(0) | | |
| **Mean(SD)** | -33.84(49.60) | 3.61(113.41) | 177.58(388.03) | | |
| **Min, Max** | -88.76,151.52 | -92.25,676.18 | -64.44,2656.4 | | |
| **Md(Q3-Q1)** | -48.16(51.23) | -22.23(79.38) | 71.40(171.36) | | |

| | | | | | |
|---|---|---|---|---|---|
| **Note: The value detected before medical treatment served as the baseline for PR and SD, the value detected at the time of minimal size of tumor served as the baseline for PD.** | | | | | |

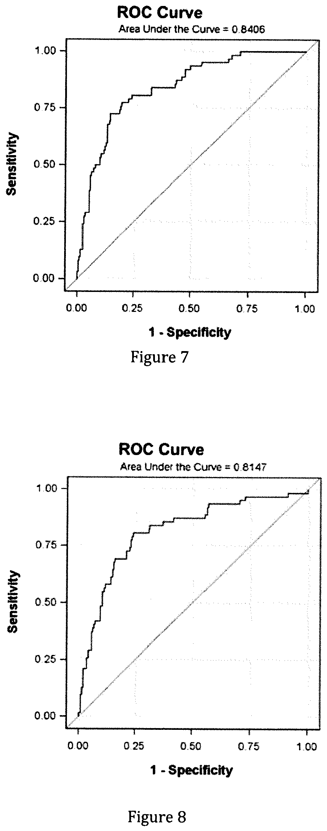

Figure 7 shows the ROC curve for PD diagnosis according to the percentage of changes.

**Table 8.3.3.2.1 Fourfold table for PD diagnosis according to the percentage of changes**

| **Index** | **Evaluation** | | |
|---|---|---|---|
| | PD | PR+SD | Total |
| >25 | | | |
| PD | 45(72.58%) | 37(17.37%) | 82(29.82%) |
| PR+SD | 17(27.42%) | 176(82.63%) | 193(70.18%) |
| Total | 62 | 213 | 275 |

| >30 | | | |
|---|---|---|---|
| PD | 45(72.58%) | 35(16.43%) | 80(29.09%) |
| PR+SD | 17(27.42%) | 178(83.57%) | 195(70.91) |
| Total | 62 | 213 | 275 |

| >34.17 | | | |
|---|---|---|---|
| PD | 45(72.58%) | 31(14.55%) | 76(27.64%) |
| PR+SD | 17(27.42%) | 182(85.45%) | 199(72.36%) |
| Total | 62 | 213 | 275 |

| >40 | | | |
|---|---|---|---|
| PD | 39(62.90%) | 28(13.15%) | 67(24.36%) |
| PR+SD | 23(37.10%) | 185(86.85%) | 208(75.64%) |
| Total | 62 | 213 | 275 |

| >45 | | | |
|---|---|---|---|
| PD | 37(59.68%) | 28(13.15%) | 65(23.64%) |
| PR+SD | 25(40.32%) | 185(86.85%) | 210(76.36%) |
| Total | 62 | 213 | 275 |

**Table 8.3.3.2.2 Diagnosis results of disease progression according to the percentage of changes**

| **Percentage of changes PD vs. PR+SD** | **TP** | **TN** | **FP** | **FN** | **Sensitivity** | | **Specificity** | | **Accuracy(%)** | **PPV(%)** | **NPV(%)** | **LR+** | **LR-** | **DOR** |
|---|---|---|---|---|---|---|---|---|---|---|---|---|---|---|
| | | | | | % | %95CI | % | %95CI | | | | | | |
| >25 | 45 | 176 | 37 | 17 | 72.58 | 61.48,83.68 | 82.63 | 77.54,87.72 | 80.36 | 54.88 | 91.19 | 4.18 | 0.33 | 12.67 |
| >30 | 45 | 178 | 35 | 17 | 72.58 | 61.48,83.68 | 83.57 | 78.59,88.54 | 81.09 | 56.25 | 91.28 | 4.42 | 0.33 | 13.39 |
| >34.17 | 45 | 182 | 31 | 17 | 72.58 | 61.48,83.68 | 85.45 | 80.71,90.18 | 82.55 | 59.21 | 91.46 | 4.99 | 0.32 | 15.59 |
| >40 | 39 | 185 | 28 | 23 | 62.9 | 50.88,74.93 | 86.85 | 82.32,91.39 | 81.45 | 58.21 | 88.94 | 4.78 | 0.43 | 11.12 |
| >45 | 37 | 185 | 28 | 25 | 59.68 | 47.47,71.89 | 86.85 | 82.32,91.39 | 80.73 | 56.92 | 88.1 | 4.54 | 0.46 | 9.87 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

Figure 8 shows the ROC curve for PD diagnosis based on the changes in Hsp90α concentration.

**Table 8.3.3.3.1 Fourfold table of PD determination based on the changes in Hsp90α concentration**

| **Index** | **Efficacy evaluation** | | |
|---|---|---|---|
| | PD | PR+SD | Total |
| >1 | | | |
| PD | 50(80.65%) | 59(27.70%) | 109(39.64%) |
| PR+SD | 12(19.35%) | 154(72.30%) | 166(60.36%) |
| Total | 62 | 213 | 275 |

| >5 | | | |
|---|---|---|---|
| PD | 50(80.65%) | 55(25.82%) | 105(38.18%) |
| PR+SD | 12(19.35%) | 158(74.18%) | 170(61.82%) |
| Total | 62 | 213 | 275 |

| >7.84 | | | |
|---|---|---|---|
| PD | 49(79.03%) | 51(23.94%) | 100(36.36%) |
| PR+SD | 13(20.97%) | 162(76.06%) | 175(63.64%) |
| Total | 62 | 213 | 275 |

| >10 | | | |
|---|---|---|---|
| PD | 47(75.81%) | 49(23.00%) | 96(34.91%) |
| PR+SD | 15(24.19%) | 164(77.00%) | 179(65.09%) |
| Total | 62 | 213 | 275 |

| >15 | | | |
|---|---|---|---|
| PD | 45(72.58%) | 46(21.60%) | 91(33.09%) |
| PR+SD | 17(27.42%) | 167(78.40%) | 184(66.91%) |
| Total | 62 | 213 | 275 |

**Table 8.3.3.3.2 PD diagnosis based on the trends of the change of cut-off values**

| **PD vs. PR+SD** | **TP** | **TN** | **FP** | **FN** | **Sensitivity** | | **Specificity** | | **Accuracy(%)** | **PPV(%)** | **NPV(%)** | **LR+** | **LR-** | **DOR** |
|---|---|---|---|---|---|---|---|---|---|---|---|---|---|---|
| | | | | | % | %95CI | % | %95CI | | | | | | |
| >1 | 50 | 154 | 59 | 12 | 80.65 | 70.81,90.48 | 72.3 | 66.29,78.31 | 74.18 | 45.87 | 92.77 | 2.91 | 0.27 | 10.78 |
| >5 | 50 | 158 | 55 | 12 | 80.65 | 70.81,90.48 | 74.18 | 68.30,80.06 | 75.64 | 47.62 | 92.94 | 3.12 | 0.26 | 12 |
| >7.84 | 49 | 162 | 51 | 13 | 79.03 | 68.90,89.17 | 76.06 | 70.33,81.79 | 76.73 | 49 | 92.57 | 3.3 | 0.28 | 11.79 |
| >10 | 47 | 164 | 49 | 15 | 75.81 | 65.15,86.47 | 77 | 71.34,82.65 | 76.73 | 48.96 | 91.62 | 3.3 | 0.31 | 10.65 |
| >15 | 45 | 167 | 46 | 17 | 72.58 | 61.48,83.68 | 78.4 | 72.88,83.93 | 77.09 | 49.45 | 90.76 | 3.36 | 0.35 | 9.6 |

| | | | | | | | | | | | | | | |
|---|---|---|---|---|---|---|---|---|---|---|---|---|---|---|
| Note: TP, true positive; TN, true negative; FP, false positive; FN, false negative; PPV, positive prediction value; NPV, negative prediction value; LR+, positive likelihood ratio; LR-, negative likelihood ratio; DOR, diagnostic accuracy odds ratio; | | | | | | | | | | | | | | |

### Example 4

### I: The assessment of anticoagulant interference (to demonstrate that EDTA-K2 does not influence Hsp90α quantification).

### Materials

Hsp90α quantitative kit, EDTA - K2, 1 set of calibrator.

### Methods

EDTA-K2 solution of 300 mg/mL was prepared. 4 uL EDTA-K2 solution was added into Hsp90α solutions (1 mL for each) in which the concentration of Hsp90α was 400 ng/mL and 200 ng/mL, respectively. The final concentration of anticoagulant was 3 mg/mL. Meanwhile, anticoagulant-free solutions at the same concentrations of Hsp90α in parallel were prepared to serve as controls. Hsp90α concentrations in different samples, with or without anticoagulant, were detected, with 6 repeats for each concentration. The average values were shown in the table below.

**Results:**

| **Concentration (ng/mL)** | **Without anticoagulant (ng/mL)** | **With anticoagulant (ng/mL)** | **Deviation (%)** |
|---|---|---|---|
| 400 | 328 | 338 | 2.9 |
| 200 | 200 | 224 | 12.2 |

Conclusion: Anticoagulant EDTA-K2 did not affect the test results of the Hsp90α quantitative kit.

### II: The assessment of interference from different blood collection tubes.

### Materials

Hsp90α kit, EDTA-K2 blood collection tube (plasma tube), EDTA-K3 blood collection tube (plasma tube), serum tube.

### Methods

Blood was collected from each person using different blood collection tubes. Hsp90α concentration of each sample was detected using the kit, with 2 repeats for each sample. The difference of the values from different blood collection tubes was calculated.

### Results:

### 1. Comparison of EDTA-K2 blood collection tube with EDTA-K3 blood collection tube.

5 healthy persons and 4 cancer patients were recruited.

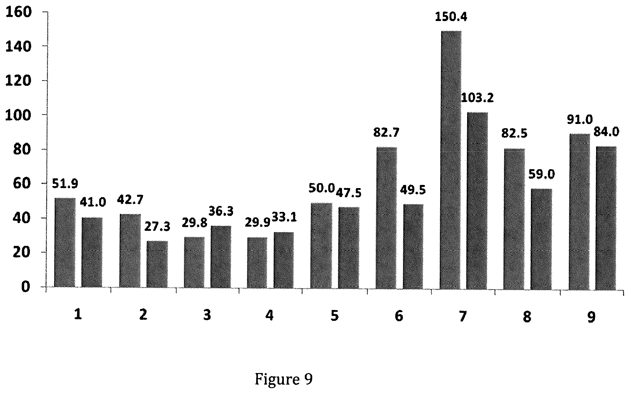

Fig. 9: Comparison of EDTA-K2 blood collection tube and EDTA-K3 blood collection tube.

Conclusion: In healthy people, the average value of Hsp90α concentration in samples from EDTA-K3 blood collection tubes was 4.37% lower than that from EDTA-K2 tubes. In cancer patients, the average value of Hsp90α concentration in samples from EDTA-K3 tubes was 26.95% lower than that from EDTA-K2 tubes. Therefore, the collection of samples using an EDTA-K3 blood collection tube would result in a decrease of Hsp90α detection value in positive samples.

### 2: Comparison of plasma tube and serum tube

148 non-cancerous disease patients were recruited.

| **Number of patients** | **Average value from plasma tubes (ng/mL)** | **Average value from serum tubes (ng/mL)** | **Deviation (%)** |
|---|---|---|---|
| 148 | 34.06 | 24.59 | 27.8 |

Conclusion: In 148 non-cancerous disease patients, the average concentration of serum Hsp90α was 27.8% lower than that of plasma Hsp90α. If a serum sample was used, the cut-off value for positive determination would be decreased.

### 3: Comparison of EDTA-K2 blood collection tube and heparin blood collection tube.

30 non-cancerous disease patients were recruited.

| **Number of patients** | **Average value from EDTA-K2 tube (ng/mL)** | **Average value of heparin tube (ng/mL)** | **Deviation (%)** |
|---|---|---|---|
| 30 | 36.07 | 43.26 | 19.9 |

Conclusion: In 30 non-cancerous disease patients, the average concentration of Hsp90α from samples collected by heparin tube was 19.9% higher than that from EDTA-K2 tube. If a blood sample was collected using a heparin tube, the cut-off value for positive determination would be increased.

### Example 5

The performance of Hsp90α quantitative detection kit in the diagnosis of liver cancer was evaluated by non-dynamic monitoring, i.e. comparing the result from a single test with pathological and clinical diagnosis results. The performance of Hsp90α quantitative detection kit in the evaluation of treatment efficacy of liver cancer was evaluated by dynamic monitoring, i.e. continuously monitoring the changes of plasma Hsp90α concentration in liver cancer patients during a clinical treatment.

### I. Design of the trials

### 1.Requirements of the trials

The study adopted blind method. Samples were detected by Hsp90α quantitative detection kit to measure Hsp90α concentration without knowing the specific information such as group in advance. Results were analyzed, and statistical indexes were calculated in terms of the level at which the detection results accord with or differ from the pathological diagnostic results, clinical diagnostic results, and the evaluation of treatment efficacy. Then the clinical performance of Hsp90α quantitative detection kit was evaluated by these indexes.

### 2. Sample collection, storage and transportation

Studied samples included non-dynamic monitoring samples and dynamic monitoring samples. Non-dynamic monitoring samples were residual plasma samples from routine clinical detections. Dynamic monitoring samples were plasma samples regularly taken from recruited patients or residual samples from regular routine detections.

The collection and storage of all plasma samples must follow the instructions of Hsp90α quantitative detection kit. Venous blood was collected and stored in EDTA-K2 anticoagulation tube. The tubes were overturned 8 to 10 times and plasma was separated by centrifugation (800-1000 g, 10 min). Aliquot of no less than 250 ul plasma per tube was added into each EP tube, and quickly placed at -18 °C for storage.

### 3. Standard for subject selection

### 3.1 Selection of non-dynamic monitoring group

Recruited patients included liver cancer group and non-liver cancer group.
(1) Liver cancer group: liver cancer patients who had been confirmed by pathological diagnosis, including different types and different stages of liver cancer.
(2) Non-liver cancer group: people who had been confirmed to have no liver cancer by biochemical, imaging or pathological methods, including healthy people without apparent disease symptom and patients having a non-cancerous liver disease, such as hepatitis and hepatocirrhosis (i.e. patients having a benign liver disease which is not liver cancer).

Healthy people: people without any known benign or malignant disease, with normal appearance, without any visible disease symptom, with normal clinical biochemical test results.

Patients with non-cancerous liver diseases should be confirmed by imaging or pathological methods as having no liver cancer.

### 3.2 Selection of dynamic monitoring group

Liver cancer patients who received a surgical treatment or medical treatment were continuously monitored to detect the relevance between plasma Hsp90α concentration and disease development.

The time points for plasma sample collection were:
(1) Dynamic monitoring of liver cancer patients who received a surgical treatment: one plasma sample was collected within 3 days before surgery, and another plasma sample was collected within 14 days after surgery.
(2) Dynamic monitoring of liver cancer patients who received a medical treatment: one plasma sample was collected before treatment; and one plasma sample was collected after each treatment cycle. Sample collection should be continued at least until the completion of the first efficacy evaluation.

### 3.3 Sample exclusion criteria

(1) In dynamic monitoring, samples from women who are pregnant, breast-feeding, or fertile but without taking a contraceptive measure during the trial;
(2) Samples from patients who are receiving adjuvant therapy after surgery;
(3) Samples from patients who are receiving radiotherapy;
(4) Patients with other types of cancer.

### II Clinical evaluation standard

1. In non-dynamic monitoring, referring to pathological gold standard to evaluate the specificity, sensitivity and accuracy of the test.
2. In dynamic monitoring, referring to CT results to evaluate the specificity, sensitivity and accuracy of the detection (i.e., to detect the relevance between plasma Hsp90α concentration and treatment efficacy).

### III Statistical analysis

Quantification data were shown as means ± S.D. Counting data were described in the number of cases and percentage.

Suitable statistical analysis method, such as t test, chi-square test, Rank-sum test and exact probability, was selected based on the type of data.

Sensitivity and specificity were used as axes to draw ROC curve and the area under ROC curve and 95% CI were calculated to evaluate the diagnosis performance.

Consistency was compared, and the percentage of consistent positive and negative cases was calculated.

SPSS software was used to analyze the Hsp90α concentrations obtained from clinical samples so as to obtain the relevance between Hsp90α and diseases, and work out a linear regression analysis (ROC curve).

### IV Results of clinical research

### 1. Statistical results of non-dynamic monitoring samples

(1) Composition of non-dynamic monitoring samples was shown in Table 9.1

**Table 9.1 Composition of non-dynamic monitoring samples**

| **Composition** | **Number of Cases** |
|---|---|
| Liver cancer patients | 602 |
| Non-cancerous liver disease patients | 151 |
| Healthy people | 592 |
| Total | 1345 |

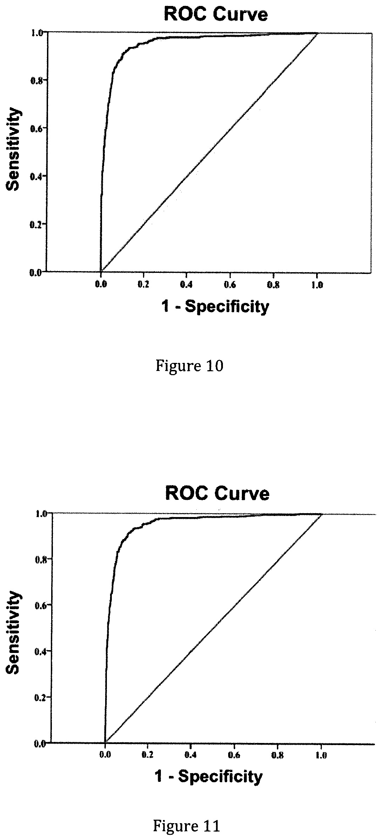

(2) ROC curve (liver cancer patients relative to healthy people), see figure 10
(3) Diagnostic index (liver cancer patients relative to healthy people), see table 9.2.

**Table 9.2 Diagnostic indexes derived from ROC curve (liver cancer patients relative to healthy people)**

| **Variable** | **Value** | |
|---|---|---|
| Cut-off | 52.79 ng/ml | 62.20 ng/ml |
| Area under curve | 0.958 | 0.958 |
| Sensitivity | 93.7 % | 91.4 % |
| Specificity | 85 % | 90 % |
| Accuracy | 94.5 % | 92.2 % |

(4) ROC curve (liver cancer patients relative to non-cancerous liver disease patients), see figure 11.
(5) Diagnostic index (liver cancer patients vs. non-cancerous liver disease patients), table 9.3

**Table 9.3 Diagnostic indexes from ROC curve (liver cancer patients vs. non-cancerous liver disease patients)**

| **Variable** | **Value** | |
|---|---|---|
| Cut-off | 63.66 ng/ml | 85.24 ng/ml |
| Area under curve | 0.958 | 0.958 |
| Sensitivity | 90.7 % | 80.7 % |
| Specificity | 90 % | 95 % |
| Accuracy | 90.3 % | 88.6 % |

(6) Statistical results of dynamic monitoring of patients who received a surgery (69 cases), table 9.4.

**Table 9.4 Changes of plasma Hsp90α concentration before and after surgery**

| **Index** | **Results** |
|---|---|
| Before surgery | |
| N | 69 |
| Mean | 183.59 |
| Min, Max | 26.54,506.00 |

| After surgery | |
|---|---|
| N | 69 |
| Mean | 118.56 |
| Min, Max | 20.00,233.00 |

| Before surgery - after surgery | |
|---|---|
| N | 69 |
| Mean | 65.04 |
| Paired t-test (P value) | 5.03(<0.001) |

As the results showed, plasma Hsp90α concentration significantly decreased after surgery, and the results were statistically significant.
(7) Disease monitoring and treatment efficacy evaluation of liver cancer patients who received an interventional therapy (9 cases)
9 patients were treated with interventional therapy, and 4 of them took benefit from the treatment (patient number 001 to 004) and decreased plasma Hsp90α concentrations were observed. Disease progressed in 5 patients and increased plasma Hsp90α concentrations were observed. These results indicated that plasma Hsp90α concentration was well correlated with disease progression. See table 9.5.

**Table 9.5 Treatment efficacy evaluation of interventional therapy in liver caner patients**

| **Patient number** | **Hsp90α concentration before treatment (ng/ml)** | **Hsp90α concentration after treatment (ng/ml)** |
|---|---|---|
| 001 | 63 | 35 |
| 002 | 184 | 128 |
| 003 | 122 | 66 |
| 004 | 133 | 77 |
| 005 | 256 | 400 |
| 006 | 130 | 226 |
| 007 | 47 | 109 |
| 008 | 31 | 66 |
| 009 | 99 | 108 |

### Example 6

The performance of Hsp90α quantitative detection kit in diagnosis of colorectal cancer was evaluated by comparing with pathological and clinical diagnosis results. The performance of Hsp90α quantitative detection kit in treatment efficacy evaluation of colorectal cancer was evaluated by dynamic monitoring of patients, namely continuously monitoring the changes of plasma Hsp90α concentrations in colorectal cancer patients during clinical therapy.

### I. Overall design of the trials

Refer to the corresponding contents in example 5. The indication was changed to colorectal cancer. Non-cancerous colorectal diseases included colorectal polyp and inflammation.

### II Standard of clinical evaluation

Refer to the corresponding contents in example 5.

### III Method of statistical analysis

Refer to the corresponding contents in example 5.

### IV Results of clinical study

### 1. Statistical results of non-dynamic monitoring samples

(1) Composition of non-dynamic monitoring samples, Table 10.1

**Table 10.1 Composition of non-dynamic monitoring samples**

| **Patients** | **Number of Cases** |
|---|---|
| Colorectal cancer patients | 475 |
| Healthy people and non-cancerous colorectal disease patients | 592 |
| Total | 1067 |

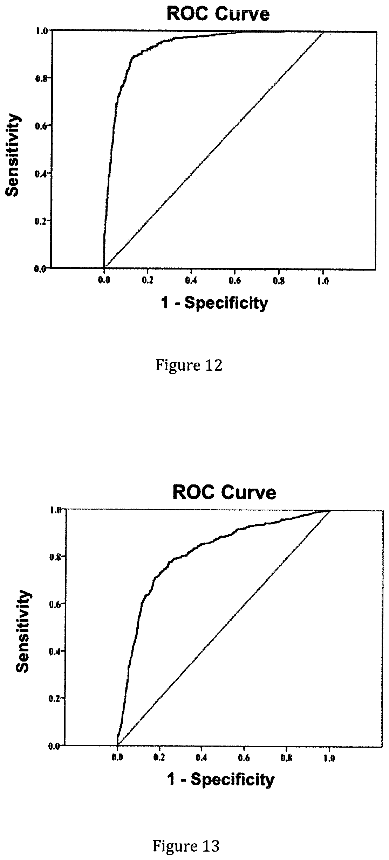

(2) ROC curve (colorectal cancer patients vs. healthy people and non-cancerous colorectal disease patients), figure 12.
(3) Diagnosis evaluation index (colorectal cancer patients vs. healthy people and non-cancerous colorectal disease patients), table 10.2

**Table 10.2 Diagnosis evaluation index from ROC curve (colorectal cancer patients vs. healthy people and non-cancerous colorectal disease patients)**

| **Index** | **Results** | |
|---|---|---|
| Cut-off | 52.79 ng/ml | 62.20 ng/ml |
| Area under curve | 0.936 | 0.936 |
| Sensitivity | 89.9% | 83.4% |
| Specificity | 85% | 90% |
| Accuracy | 87% | 87% |

(4) Statistical results of dynamic monitoring of patients receiving surgery (101 cases), table 10.3

**Table 10.3 Plasma Hsp90α concentrations before and after surgery**

| **Index** | **Results** |
|---|---|
| Before surgery | |
| N | 101 |
| Mean | 145.84 |
| Min, Max | 50.72, 554.40 |

| After surgery | |
|---|---|
| N | 101 |
| Mean | 94.08 |
| Min, Max | 66.19, 105.63 |

| Before surgery - after surgery | |
|---|---|
| N | 101 |
| Mean | 51.76 |
| Paired t test (P value) | 5.85(<0.001) |

As the results showed, plasma Hsp90α concentration significantly decreased after surgery, and the results were statistically significant.
(5) 10 patients took benefit (CR+PR+SD) from medical treatment, and treatment efficacy evaluation results showed that 9 of them had decreased plasma Hsp90α concentrations. The detection results of Hsp90α correlated well with the treatment efficacy. (Table 10.4)

**Table 10.4 Detection results from colorectal patients who obtained clinical benefit from medical treatment**

| **Patient number** | **Hsp90α concentration before chemotherapy (ng/ml)** | **Hsp90α concentration after chemotherapy (ng/ml)** |
|---|---|---|
| 001 | 221 | 112 |
| 002 | 41 | 36 |
| 003 | 49 | 20 |
| 004 | 171 | 69 |
| 005 | 46 | 29 |
| 006 | 125 | 66 |
| 007 | 37 | 40 |
| 008 | 24 | 20 |
| 009 | 106 | 26 |
| 010 | 32 | 31 |

### Example 7

The performance of Hsp90α quantitative detection kit in diagnosis of breast cancer was evaluated by comparing with pathological and clinical diagnosis results. The performance of Hsp90α quantitative detection kit in treatment efficacy evaluation of breast cancer was evaluated by dynamic monitoring of patients, namely continuously monitoring the changes of plasma Hsp90α concentration in breast cancer patients during clinical therapy.

### I. Overall design of the trials

Refer to the corresponding contents in example 5. The indication was changed to breast cancer. Non-cancerous breast diseases included mastitis and hyperplasia of mammary glands.

### II Standard of clinical evaluation

Refer to the corresponding contents in example 5.

### III Method of statistical analysis

Refer to the corresponding contents in example 5.

### IV Results of clinical study

### 1. Statistical results of non-dynamic monitoring samples

(1) Composition of non-dynamic monitoring samples, Table 11.1

**Table 11.1, Composition of non-dynamic monitoring samples**

| **Groups** | **Number of Cases** |
|---|---|
| Breast cancer patients | 800 |
| Non-cancerous breast disease patients | 172 |
| Healthy people | 592 |
| Total | 1564 |

(2) ROC curve (breast cancer patients vs. healthy people), figure 13.
(3) Diagnostic index (breast cancer patients vs. healthy people), table 11.2.

**Table 11.2 Diagnostic index derived from ROC curve (breast cancer patients vs. healthy people)**

| **Index** | **Results** | |
|---|---|---|
| Cut-off | 52.66 ng/ml | 61.92 ng/ml |
| Area under curve | 0.817 | 0.817 |
| Sensitivity | 65.6% | 54.6% |
| Specificity | 85% | 90% |
| Accuracy | 73.85% | 69.68% |

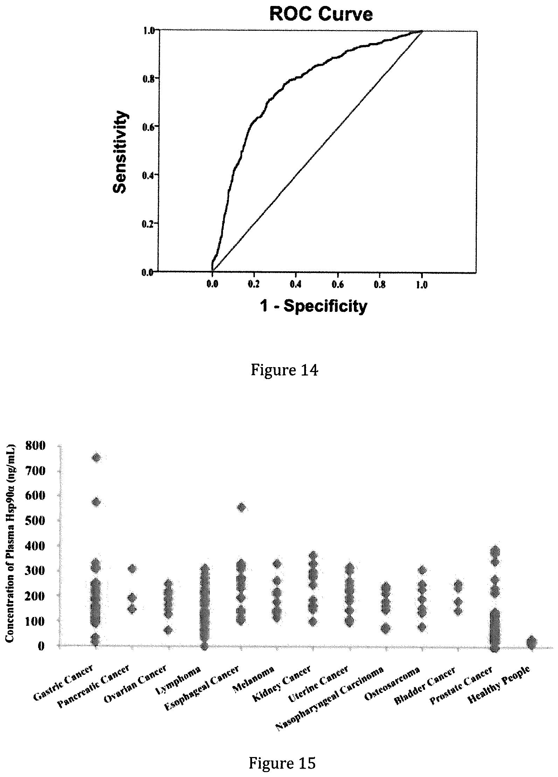

(4) ROC curve (breast cancer patients vs. non-cancerous breast disease patients), figure 14.
(5) Diagnostic index (breast cancer patients vs. non-cancerous breast disease patients), table 11.3.

**Table 11.3 Diagnostic index derived from ROC curve (breast cancer patients vs. non-cancerous breast disease patients)**

| **Variable** | **Value** | |
|---|---|---|
| Cut-off | 63.41ng/ml | 72.21ng/ml |
| Area under curve | 0.769 | 0.769 |
| Sensitivity | 52.4% | 41.9% |
| Specificity | 84.9% | 90.1% |
| Accuracy | 68.29% | 65.41% |

(6) Statistical results of dynamic monitoring of patients who received surgery (26 cases), table 11.4.

**Table 11.4 Plasma Hsp90α concentration before and after surgery**

| **Index** | **Results** |
|---|---|
| Before surgery | |
| N | 26 |
| Mean | 75.19 |
| Min, Max | 39.50,168.14 |

| After surgery | |
|---|---|
| N | 26 |
| Mean | 45.33 |
| Min, Max | 20.00,188.54 |

| Before surgery minus after surgery | |
|---|---|
| N | 26 |
| Mean | 29.86 |
| paired t-test (P value) | 4.065(<0.001) |

(7) The results of dynamic monitoring of breast cancer patients who took benefit (CR+PR+SD) from chemotherapy (18 cases) showed that plasma Hsp90α concentrations decreased in these patients, and the results were statistically significant. (Table 11.5)

**Table 11.5 Detection results of breast cancer patients taking benefit from chemotherapy**

| | **Index** | **Results** |
|---|---|---|
| **Hsp90α concentration** before chemotherapy (ng/ml) | Mean | 103 |
| **Hsp90α concentration** after chemotherapy (ng/ml) | Mean | 81 |
| Δ=Before chemotherapy - after chemotherapy (ng/ml) | Mean | 22 |
| | Paired t value | 2.338 |
| | P value | 0.03 |

### Example 8

The performance of Hsp90α quantitative detection kit in diagnosis of multiple types of cancer was evaluated by comparing with pathological and clinical diagnosis results. As the results showed, when compared with healthy people, patients suffering from gastric cancer, pancreatic cancer, ovarian cancer, lymphoma, esophageal cancer, melanoma, renal cancer, uterine cancer, nasopharyngeal cancer, osteosarcoma, bladder cancer, prostate cancer and other types of cancer had significantly increased level of plasma Hsp90α concentration (figure 15), and the results were statistically significant. The average Hsp90α concentrations of patients suffering from different types of cancer were shown in table 12.1.

**Table 12.1 Average of plasma Hsp90α concentration in different types of cancer**

| **Type of cancer** | **Cases** | **Average (ng/ml)** |
|---|---|---|
| Gastric cancer | 55 | 199.70 |
| Pancreatic cancer | 5 | 199.73 |
| Ovarian cancer | 10 | 180.47 |
| Lymphoma | 42 | 161.30 |
| Esophageal cancer | 20 | 239.13 |
| Melanoma | 10 | 190.11 |
| Renal cancer | 14 | 242.00 |
| Uterine cancer | 15 | 209.77 |
| Nasopharyngeal cancer | 8 | 168.65 |
| Osteosarcoma | 8 | 188.68 |
| Bladder cancer | 4 | 206.56 |
| Prostate cancer | 117 | 69.12 |

## Claims

1. A method of determining whether a subject is suffering from a cancer or has a risk of cancer, including detecting the concentration of Hsp90α (heat shock protein 90α) in a plasma sample from the subject, wherein if the concentration reaches or exceeds a pre-set threshold, it is determined that the subject is suffering from a cancer or has a risk of cancer; wherein the threshold is selected from the range of 50 ng/ml to 120 ng/ml, and wherein the concentration of Hsp90α is detected using EDTA-K2 as an anticoagulant.

2. The method of claim 1 further comprising detecting the concentration of at least one other tumor biomarker in said sample, wherein if the concentration of Hsp90α and the at least one other tumor biomarker reaches or exceeds a pre-set threshold, it is determined that the subject is suffering from a cancer or has a risk of cancer.

3. The method of any one of claims 1-2, wherein the threshold is selected from the group consisting of 50, 53, 56, 62, 63, 64, 67, 72, 82, 85, and 117 ng/ml.

4. The method of any one of claims 1-3, wherein the threshold is 82 ng/ml.

5. The method of any one of claims 1-4, wherein the cancer is selected from the group consisting of lung cancer, liver cancer, colorectal cancer, and breast cancer.

6. A kit for determining whether a subject is suffering from a cancer or has a risk of cancer including: a monoclonal antibody against human secretory Hsp90α,
a detection reagent,
a container for receiving a plasma sample, wherein the container comprises EDTA-K2 as an anticoagulant,
and instructions, wherein the instructions indicate the threshold of Hsp90α concentration in a plasma sample of a healthy human, and wherein if the Hsp90α concentration in a plasma sample from the subject reaches or exceeds the threshold, the subject is determined to have a cancer or have a risk of cancer; wherein the threshold is selected from the range of 50 ng/ml to 120 ng/ml, and wherein the concentration of Hsp90α is detected using EDTA-K2 as an anticoagulant.

## Patentansprüche

1. Verfahren zur Feststellung, ob eine Testperson an Krebs erkrankt ist oder ein Krebsrisiko hat, beinhaltend die Bestimmung der Konzentration von Hsp90α (Hitzeschockprotein 90α) in einer Plasmaprobe von der Testperson, wobei festgestellt wird, dass die Testperson an Krebs erkrankt ist oder ein Krebsrisiko hat, wenn die Konzentration einen vorgegebenen Grenzwert erreicht oder überschreitet; wobei der Grenzwert ausgewählt wird aus einem Bereich von 50 ng/ml bis 120 ng/ml, und wobei die Konzentration von Hsp90α unter Verwendung von EDTA-K2 als Antikoagulans bestimmt wird.

2. Verfahren gemäß Anspruch 1, weiterhin umfassend die Bestimmung der Konzentration mindestens eines weiteren Tumorbiomarkers in der besagten Probe, wobei festgestellt wird, dass die Testperson an Krebs erkrankt ist oder ein Krebsrisiko hat, wenn die Konzentration des Hsp90α und des mindestens einen weiteren Tumorbiomarkers einen vorgegebenen Grenzwert erreicht oder überschreitet.

3. Verfahren gemäß einem der Ansprüche 1-2, wobei der Grenzwert ausgewählt wird aus der Gruppe bestehend aus 50, 53, 56, 62, 63, 64, 67, 72, 82, 85 und 117 ng/ml.

4. Verfahren gemäß einem der Ansprüche 1-3, wobei der Grenzwert 82 ng/ml ist.

5. Verfahren gemäß einem der Ansprüche 1-4, wobei der Krebs ausgewählt wird aus der Gruppe bestehend aus Lungenkrebs, Leberkrebs, Darmkrebs und Brustkrebs.

6. Kit zur Bestimmung, ob eine Testperson an Krebs erkrankt ist oder ein Krebsrisiko hat, enthaltend: einen monoklonalen Antikörper gegen menschliches sekretorisches Hsp90α,
ein Bestimmungsreagenz,
einen Behälter für die Aufnahme einer Plasmaprobe, wobei der Behälter EDTA-K2 als Antikoagulans umfasst,
und eine Anleitung, wobei die Anleitung den Grenzwert der Hsp90-Konzentration in einer Plasmaprobe eines gesunden Menschen angibt, und wobei festgestellt wird, dass die Testperson an Krebs erkrankt ist oder ein Krebsrisiko hat, wenn die Hsp90α-Konzentration in einer Plasmaprobe von der Testperson den Grenzwert erreicht oder überschreitet; wobei der Grenzwert ausgewählt ist aus dem Bereich von 50 ng/ml bis 120 ng/ml, und wobei die Konzentration von Hsp90α unter Verwendung von EDTA-K2 als Antikoagulans bestimmt wird.

## Revendications

1. Procédé permettant de déterminer si un sujet est atteint d'un cancer ou s'il présente un risque de cancer, comprenant la détection de la concentration en Hsp90α (protéine de choc thermique 90α) dans un échantillon de plasma provenant du sujet, dans lequel, si la concentration atteint ou dépasse un seuil prédéterminé, il est établi que le sujet est atteint d'un cancer ou présente un risque de cancer ; dans lequel le seuil est choisi dans la plage entre 50 ng / ml et 120 ng / ml, et dans lequel la concentration en Hsp90α est détectée en utilisant de l'EDTA-K2 en tant qu'anticoagulant.

2. Procédé selon la revendication 1, comprenant en outre la détection de la concentration d'au moins un autre marqueur biologique de tumeur dans ledit échantillon, dans lequel, si la concentration en Hsp90α et en l'au moins un autre marqueur biologique de tumeur atteint ou dépasse un seuil prédéterminé, il est établi que le sujet est atteint d'un cancer ou présente un risque de cancer.

3. Procédé selon l'une quelconque des revendications 1 à 2, dans lequel le seuil est choisi à partir du groupe constitué par les concentrations 50, 53, 56, 62, 63, 64, 67, 72, 82, 85, et 117 ng / ml.

4. Procédé selon l'une quelconque des revendications 1 à 3, dans lequel le seuil est de 82 ng / ml.

5. Procédé selon l'une quelconque des revendications 1 à 4, dans lequel le cancer est sélectionné dans le groupe constitué par le cancer du poumon, le cancer du foie, le cancer colorectal, et le cancer du sein.

6. Trousse destinée à déterminer si un sujet est atteint d'un cancer ou s'il présente un risque de cancer, comprenant : un anticorps monoclonal contre la protéine Hsp90α humaine sécrétée,
un réactif de détection,
un récipient destiné à recevoir un échantillon de plasma, dans laquelle le récipient comprend de l'EDTA-K2 en tant qu'anticoagulant,
et des instructions, dans laquelle les instructions précisent le seuil de la concentration en Hsp90α dans un échantillon de plasma pour un être humain en bonne santé, et dans laquelle, si la concentration en Hsp90α dans un échantillon provenant du sujet atteint ou dépasse le seuil, il est établi que le sujet est atteint d'un cancer ou présente un risque de cancer ; dans laquelle le seuil est choisi dans la plage entre 50 ng / ml et 120 ng / ml, et dans laquelle la concentration en Hsp90α est détectée en utilisant de l'EDTA-K2 en tant qu'anticoagulant.
